# EUROPEAN PATENT APPLICATION

(11) **EP 4 570 263 A1**
(43) Date of publication of application: **18.06.2025**
(21) Application number: 23217230.4
(22) Date of filing: 15.12.2023
(51) Int. Cl.: A61K 39/00, C07K 14/725, C07K 14/74, A61K 38/00

(54) **TCRS RECOGNIZING G12V MUTATED RAS AND USES THEREOF**

(71) Applicant: Genovie AB, 151 36 Södertälje (SE)
(72) Inventor: JARVIS, Reagan, 151 36 Södertälje (SE); PASE, Luke, 151 36 Södertälje (SE); HILL, Ryan, 151 36 Södertälje (SE); SALTER, Hugh, 151 36 Södertälje (SE)
(74) Representative: Aera A/S

(57) **Abstract**

The invention relates to a selection of verified targets for T-cell receptors (TCR) against mutated human rat sarcoma (RAS) peptides as well as to several new T-cell receptors (TCR), which are isolated and/or expressed in an engineered cell, and which specifically recognize a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, which consists of VWGAVGVGK (SEQ ID NO: 1) or VVGAVGVGK (SEQ ID NO:2) presented in a HLA-A*11 complex, and wherein the individual TCR comprises a paired TCR alpha- and TCR beta-chain.

The invention further relates to engineered cells and vectors expressing said TCRs and medical uses of the same for treating cancer patients.

## Description

### Technical field

The present invention relates to the field of treating cancer patients who express a mutated RAS variant. Herein disclosed is a selection of verified targets for T-cell receptors (TCR) against mutated human rat sarcoma (RAS) peptides, as well as novel T-cell receptors (TCR) which are isolated and/or expressed in an engineered cell, and which specifically recognize a specific G12V mutated human rat sarcoma (RAS) peptide, wherein the peptide can be a mutated human Kirsten RAS (KRAS) peptide, a mutated human Harvey RAS (HRAS) peptide, or a human neuroblastoma RAS (NRAS) peptide, and wherein the peptide consists of VVVGAVGVGK (SEQ ID NO: 1) or WGAVGVGK (SEQ ID NO:2) presented in an HLA-A*11 complex.

### Background

Adoptive cell transfer (ACT) therapies using engineered T cells (TCR-Ts) or chimeric antigen receptor (CAR)-T cells, have gained much traction in recent years. The use of CAR-T in the treatment of B cell malignancies has demonstrated to be very effective (Weber EW, Maus MV, Mackall CL. The Emerging Landscape of Immune Cell Therapies. Cell (2020) 181 :46-62), however this efficacy is not replicable in the treatment of solid tumours. TCR-T-based immunotherapy holds much promise for patients with difficult to treat cancers where there are currently no alternative treatment options. A great advantage of TCR-T-based therapy relates to T cells being able to recognise a much larger number of antigenic targets and from all cellular compartments than chimeric antigen receptors. The TCR-antigen interaction is the core component of the targeting mechanism that allows T cells to kill cancer cells. TCRs also have lower epitope density requirement for activation compared to chimeric antigen receptors. An important consideration when designing TCR-T-based therapy is the Human Leucocyte Antigen (HLA) restriction, as any given TCR can only be used to treat patients with the corresponding HLA genetic background to avoid tissue rejection. This restriction needs to be taken into consideration when developing a therapeutic TCR and when using such a TCR in engineered T cells.

There remains an unmet need in the art for obtaining specific and safe TCRs for difficult-to-treat cancers.

### The T-cell Receptor (TCR)

The TCR is the component of the T-cell responsible for interacting with and sensing the targets of T-cell adaptive immunity. In general terms, the TCR is comprised of a heterodimeric protein complex presented on the cell surface. Each of the two TCR chains are composed of two extracellular domains, being the variable (V)-region and the constant (C)-region, both of the immunoglobulin superfamily (IgSF) domain, forming antiparallel β-sheets. These are anchored in the cell membrane by a type-I transmembrane domain, which adjoins a short cytoplasmic tail. The quality of the T-cells to adapt and detect diverse molecular constituents arises from variations in the TCR chains that is generated during T-cell genesis. This variation is generated by somatic recombination in a similar manner to antibody genesis in B cells.

### TCR chain diversity

The T-cell pool consists of several functionally and phenotypically heterogeneous subpopulations. However, T-cells may be broadly classified as αβ or γδ according to the somatically rearranged TCR isoform they express at their surface. There exist two TCR chain pair isoforms; TCR alpha (TRA) and TCR beta (TRB) pairs; and TCR gamma (TRG) and TCR delta (TRD) pairs. T-cells expressing TRA:TRB pairs are referred to as αβ T-cells, while T-cells expressing TRG:TRD pairs are often referred to as γδ T-cells.

TCRs of both αβ and γδ forms are responsible for recognition of diverse ligands, or 'antigens', and each T-cell generates αβ or γδ receptor chains *de novo* during T-cell maturation. These *de novo* TCR chain pairs achieve diversity of recognition through generation of receptor sequence diversity in a process called somatic V(D)J recombination after which each T-cell expresses copies of a single distinctly rearranged TCR. At the TRA and TRG loci, a number of discrete variable (V) and functional (J) gene segments are available for recombination and juxtaposed to a constant (C) gene segments, thus referred to as VJ recombination. Recombination at the TRB and TRD loci additionally includes a diversity (D) gene segment and is referred to as VDJ recombination.

Each recombined TCR possess potential for unique ligand specificity, determined by the structure of the ligand-binding site formed by the α and β chains in the case of αβ T-cells or γ and δ chains in the case of γδ T-cells. The structural diversity of TCRs is largely confined to three short hairpin loops on each chain, called complementarity-determining regions (CDR). Three CDRs are contributed from each chain of the receptor chain pair, and collectively these six CDR loops sit at the membrane distal end of the TCR extracellular domain to form the antigen-binding site.

Sequence diversity in each TCR chain is achieved in two modes. First, the random selection of gene segments for recombination provides basal sequence diversity. For example, TRB recombination occurs between 47 unique V, 2 unique D and 13 unique J germline gene segments. In general, the V gene segment contributes both the CDR1 and CDR2 loops and are thus germline encoded. The second mode to generate sequence diversity occurs within the hypervariable CDR3 loops, which are generated by random deletion of template nucleotides and addition of non-template nucleotides, at the junctions between recombining V, (D) and J gene segments.

### TCR:CD3 Complex

Mature αβ and γδ TCR chain pairs are presented on the cell surface in a complex with accessory CD3 subunits, denoted ε, γ, δ and ζ. These subunits associate with αβ or γδ TCRs as three dimers (εγ, εδ, ζζ). This TCR:CD3 complex forms the unit for initiation of cellular signalling responses upon engagement of an αβ or γδ TCR with cognate antigen. The CD3 accessories associated as a TCR:CD3 complex contribute signalling motifs called immunoreceptor tyrosine-based activation motifs (ITAMs).

CD3ε, CD3γ and CD3δ each contribute a single ITAM while the CD3ζ homodimer contains 3 ITAMs. The three CD3 dimers (εγ, εδ, ζζ) that assemble with the TCR thus contribute 10 ITAMs. Upon TCR ligation with cognate antigen, phosphorylation of the tandem tyrosine residues creates paired docking sites for proteins that contain Src homology 2 (SH2) domains, such as the critical ζ-chain-associated protein of 70 kDa (ZAP- 70). Recruitment of such proteins initiate the formation of TCR:CD3 signalling complexes that are ultimately responsible for T-cell activation and differentiation.

### αβ T-cells

αβ T-cells are generally more abundant in humans than their γδ T-cell counterparts. A majority of αβ T-cells interact with peptide antigens that are presented by complexes on the cell surface. These complexes are referred to as Major Histocompatibility Complexes (MHC), encoded by Human Leucocyte Antigen (HLA) family of genes, for simplicity both the gene and MHC will collectively be referred to herein as HLA. Peptide-HLA (pHLA)-recognising T-cells were the first to be described and are by far the best characterised. More rare forms of αβ T-cells have also been described. Mucosal-associated invariant T (MAIT) cells appear to have a relatively limited α and β chain diversity, and recognise bacterial metabolites rather than protein fragments. The invariant natural killer T-cells (iNK T-cells) and germline-encoded mycolyl-reactive T-cells (GEM T-cells) are restricted to recognition of glycolipids that are cross-presented by non-HLA molecules. iNK T-cells are largely considered to interact with CD1d-presented glycolipids, whereas GEM T-cells interact with CD1b-presented glycolipids. Further forms of T-cells are thought to interact with glycolipids in the context of CD1a and CD1c, however, such cells are yet to be characterised in significant detail.

### Conventional αβ T-cells

The key feature of most αβ T-cells is the recognition of peptide antigens in the context of HLA molecules. These are often referred to as 'conventional' αβ T-cells. Within an individual, self-HLA molecules present peptides from self and foreign proteins to T-cells, providing the essential basis for adaptive immunity against malignancies and foreign pathogens, adaptive tolerance towards commensal organisms, foodstuffs and self. The HLA locus that encodes HLA proteins is the most gene-dense and polymorphic region of the human genome, and there are in excess of 12,000 alleles described in humans. The high degree of polymorphism in the HLA locus ensures a diversity of peptide antigen presentation between individuals, which is important for immunity at the population level.

### HLA class I and II

There are two forms of classical HLA complexes: HLA class I (HLAI) and HLA class II (HLAII). There are three classical HLAI genes: *HLA-A, HLA-B, HLA-C.* These genes encode a membrane-spanning α-chain, which associates with an invariant beta-2-microglobulin (b2M) chain. The HLAI α-chain is composed of three domains with an immunoglobulin fold: α1, α2 and α3. The α3 domain is membrane-proximal and largely invariant, while the α1 and α2 domains together form the polymorphic membrane-distal antigen-binding cleft. There are six classical HLAII genes: *HLA-DPA1, HLA-DPB1, HLA- DQA1, HLA-DQB1, HLA-DRA,* and *HLA-DRB1.* These genes encode paired DP, DQ and DR heterodimeric HLA complexes comprising a α-chain and a β-chain. Each chain has two major structural domains with an immunoglobulin fold, where the α2 and β2 domain comprise membrane-proximal and largely invariant modules similar to that of HLAI α3 domain. The HLAII α2 and β2 domains together form the membrane-distal antigen-binding cleft and are regions of high polymorphism.

The antigen-binding cleft of HLAI and HLAII comprises two anti-parallel α-helices on a platform of eight anti-parallel β-sheets. In this cleft the peptide antigen is bound and presented in an extended conformation. The peptide-contacting residues in HLAI and HLAII are the location of most of the sequence polymorphism, which constitutes the molecular basis of the diverse peptide repertoires presented by different HLA alleles. The peptide makes extensive contacts with the antigen-binding cleft and as a result each HLA allele imposes distinct sequence constraints and preferences on the presented peptides. A given peptide will thus only bind a limited number of HLAs, and reciprocally each allele only accommodates a particular fraction of the peptide collection from a given protein. The set of HLAI and HLAII alleles that is present in each individual is called the HLA haplotype. The polymorphism of HLAI and HLAII genes and the co-dominant expression of inherited alleles drives very large diversity of HLA haplotype across the human population, which when coupled to the enormous sequence diversity of αβ TCR, presents high obstacles to standardisation of analysis of these HLA-antigen-TCR interactions.

### αβ TCR engagement of HLAI and HLAII

The αβ TCR recognize peptides as part of a mixed pHLA binding interface formed by residues of both the HLA and the peptide antigen (altered self). HLAI complexes are presented on the surface of nearly all nucleated cells and are generally considered to present peptides derived from endogenous proteins. T-cells can thus interrogate the endogenous cellular proteome of an HLAI-presenting cell by sampling pHLAI complexes of an interacting cell. Engagement of HLAI requires the expression of the TCR co-receptor CD8 by the interacting T-cell, thus HLAI sampling is restricted to CD8⁺ αβ T-cells. In contrast, the surface presentation of HLAII complexes is largely restricted to professional APCs and are generally considered to present peptides derived from proteins exogenous to the presenting cell. An interacting T-cell can therefore interrogate the proteome of the extracellular microenvironment in which the presenting cell resides. The engagement of HLAII requires the expression of the TCR co-receptor CD4 by the interacting T-cell, and thus HLAII sampling is restricted to CD4⁺ αβ T-cells.

### RAS Oncogenes

Members of the RAS super-family of proteins (KRAS/NRAS/HRAS) are involved in cellular signal transduction pathways. The RAS family members have an identical amino acid sequence in positions 1 through to 86, and more than 99% of all mutations occur at positions 12, 13 or 61 (Prior IA et al., Cancer Res. 2012 May 15; 72(10): 2457-2467). As these positions are in the domains responsible for GTP dephosphorylation into GDP, the mutations lead to constitutive activation of the RAS protein. Since they are a gain of function mutations, one mutated allele expressed at normal level is sufficient to support cancer establishment and growth. Most predominant mutations in the RAS occur in KRAS (85%) but also in NRAS (11%) and HRAS (4%). KRAS mutation patterns are dominated by G to T transitions at the second base of codon 12 resulting in G12V mutation (Prior IA et al., Cancer Res. 2012 May 15; 72(10): 2457-2467). KRAS is frequently mutated in a variety of hard-to-treat cancers, including pancreatic cancer (∼90%), colon cancer (∼43%), lung cancer (∼30%), and melanoma (∼50%).

TCR-T-based immunotherapy targeted towards RAS-mutations holds much promise for patients with difficult to treat cancers where there are currently no alternative treatment options. Still, there is a need for identifying targets that are good and TCRs that recognize these suitable targets with high affinity and specificity without displaying cytotoxicity towards e.g., suboptimal targets or wild-type RAS.

### Summary of the invention

In general, the identification of TCRs that have a particularly suitable profile for therapeutic use is not straightforward and typically has a high attrition rate. The current invention thus for the first time discloses several verified suitable RAS-targets as well as TCRs that have a particularly suitable profile for therapeutic use in cancer therapy.

The current invention relates to several new T-cell receptors (TCR), which are isolated and/or expressed in an engineered cell, and which specifically recognize a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, which consists of VVVGAVGVGK (SEQ ID NO: 1) or VVGAVGVGK (SEQ ID NO:2) presented in a HLA-A*11 complex, and wherein the individual TCR comprises a paired TCR alpha- and TCR beta-chain, wherein the TCR alpha chain variable domain comprises SC1 alpha-CDR1 alpha-SC2alpha-CDR2alpha-SC3alpha-CDR3alpha-SC4alpha-SC5alpha, and the TCR beta chain variable domain comprises SC1 beta-CDR1beta-SC2 beta-CDR2 beta-SC3beta-CDR3beta-SC4beta-SC5beta, wherein SC is a scaffold region and CDR is a complementarity determining region.

A TCR according to the invention does not specifically recognize a G12V mutated human RAS peptide, consisting of VVVGAVGVGK (SEQ ID NO: 1) or WGAVGVGK (SEQ ID NO: 2) when the peptide is presented in a HLA-A*11:03 complex, and does not, when expressed in an engineered cell, cause specific cytotoxic activity towards cells expressing the wild type human RAS peptide, such as VVVGAGGVGK (SEQ ID NO: 3) or VVGAGGVGK (SEQ ID NO: 4) presented in any HLA-A complex, such as, but not limited to, a HLA-A*11 complex.

Further, when expressed in an engineered cell, a TCR according to the invention does not cause specific cytotoxic activity towards cells not expressing a G12V mutated RAS peptide, such as, but not limited to, towards cells expressing the wild type human RAS peptide, such as VVVGAGGVGK (SEQ ID NO: 3) presented in a HLA-A*11 complex or VVGAGGVGK (SEQ ID NO: 4) presented in a HLA-A*11 complex.

When expressed in an engineered cell, a TCR according to the invention does not cause cytotoxic activity towards cells expressing the G12V mutated human RAS peptide, wherein the peptide consists of VVVGAVGVGK (SEQ ID NO: 1) or WGAVGVGK (SEQ ID NO: 2) which is presented in an allelic variant of the human leukocyte antigen (HLA) molecule other than an allelic variant of the human leukocyte antigen (HLA) molecule selected from the group consisting of HLA-A*11, HLA- A*68:02, HLA- B*54:01, and HLA-A*30:01 serotype. In general, when expressed in an engineered cell, a TCR according to the invention does not cause strong cytotoxic activity towards cells expressing the G12V mutated human RAS peptide, wherein the peptide consists of VWGAVGVGK (SEQ ID NO: 1) and/or WGAVGVGK (SEQ ID NO: 2) presented in a allelic variant of the human leukocyte antigen (HLA) molecule other than the HLA-A*11 serotype.

In one embodiment, as demonstrated in the experimental section, the TCR according to the current invention is a TCR, which, when expressed in an engineered cell, has detectable cross-reactivity towards any somatically expressed HLA-presented peptide other than a G12V mutated human rat sarcoma (RAS) peptide, which consists of VVVGAVGVGK (SEQ ID NO: 1) and/or WGAVGVGK (SEQ ID NO: 2) and which is presented in a HLA-A*11 complex in a cross-reactivity assay.

A TCR disclosed herein typically comprises:
a. a TCR alpha chain CDR3 sequence with at least 80% identity to a TCR alpha chain CDR3 sequence selected from the group consisting of SEQ ID NOS: SEQ-,
   and
b. a TCR beta chain CDR3 sequence with at least 80% identity to a TCR beta chain CDR3 sequence selected from the group consisting of SEQ ID NOS:.

In embodiments, the TCR according to the current invention comprises:
c. a TCR alpha chain CDR3 sequence with a TCR alpha chain CDR3 sequence selected from the group consisting of SEQ ID NOS:,
   and
d. a TCR beta chain CDR3 sequence with a TCR beta chain CDR3 sequence selected from the group consisting of SEQ ID NOS:.

In a currently preferred embodiment of the invention the TCR alpha and beta chain variable domain scaffold regions or CDRs of the TCR comprises and/or consists of one of the sequences selected from the group consisting of (full-sequences of TCR-1-TCR-4).

A TCR according to the current invention can be selected from the group comprising chimeric, humanized and/or human TCRs.

A TCR according to the invention can comprise constant regions of the alpha and beta chains which have been modified to include cysteine residues to allow covalent connection by di-sulphide bridges or comprise no covalent connections between said paired alpha and beta chains. In embodiments, the alpha and/or beta chain comprise(s) a constant region of another mammal, such as, but not limited to, a mouse chimeric constant region(s). Further, the alpha and/or beta chain can have been modified to include cysteine residues to allow di-sulphide bridges.

In aspects, the TCR comprises a TCR alpha chain a TCR beta chain which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein. In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The current invention in one aspect relates to a soluble TCR and/or a conjugated soluble TCR according to the current invention. In aspects, the TCR is a bi-specific T-cell engager (BiTE), or it is an immune mobilizing monoclonal T-cell receptor.

The invention further relates to a pharmaceutical composition comprising a soluble protein consisting of all or a functional part of a TCR as disclosed and/or defined herein and at least one pharmaceutically acceptable excipient, carrier or stabilizer.

A pharmaceutical composition of the current invention comprises a soluble TCR, a conjugated soluble TCR, a cell expressing a TCR or a composition comprising a TCR, wherein the TCR is a TCR according to the current invention. Said soluble TCR can been conjugated to a radionuclide, a chemotherapeutic agent, a toxin or another therapeutically active component, and at least one pharmaceutically acceptable excipient, carrier or stabilizer.

In one aspect, the invention relates to a method of treating a disorder characterized by G12V muted human RAS expression in a subject comprising administering to said subject a therapeutically effective amount of genetically engineered cells that express a TCR as disclosed and/or defined herein, or a pharmaceutical composition disclosed and/or defined herein, wherein said disorder is a solid or haematological malignancy expressing the peptide of VVVGAVGVGK (SEQ ID NO: 1) and/or VVGAVGVGK (SEQ ID NO: 2), wherein said engineered cell is a T-cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour or into the volume surrounding a solid tumour.

Said method is in particular intended for treating a disorder in a subject, wherein said subject carries an HLA-A*11 allele. In addition, said method can be used for treating a said subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by the TCR according to the current invention, such as wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to the current invention.

In one aspect, the current invention relates to a TCR disclosed, described and/or defined herein for use as a medicament. In particular, the current invention relates to a TCR disclosed, described and/or defined herein for use in the treatment of a cancer in a patient carrying HLA-A*11, comprising administering to said patient said TCR or a pharmaceutical composition comprising said TCR, wherein said cancer is a solid or haematological malignancy expressing the peptide of SEQ ID NO: 1 or SEQ ID NO: 2, and wherein said TCR or a pharmaceutical composition comprising said TCR is infused systemically into said patient or injected or pumped directly into a solid tumour, into a blood vessel that feeds a tumour or into the volume surrounding a solid tumour in said patient.

In aspects, said treatment comprises administering to said patient an engineered cell, wherein said engineered cell is a T-cell, T-cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour or into the volume surrounding a solid tumour.

Typically, the use related to herein is for treating a disorder in a subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by a TCR disclosed, described and/or defined herein, such as wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to the current invention.

In one aspect, the invention relates to a nucleic acid encoding a TCR comprising a paired TCR alpha- and TCR beta-chain as disclosed, described and/or defined herein.

In another aspect, the invention relates to a vector comprising a nucleic acid encoding a TCR comprising a paired TCR alpha- and TCR beta-chain as disclosed, described and/or defined herein. Said vector can be selected from the group consisting of an expression vector, a coding vector, or a viral vector. In certain aspects, said vector further comprises a nucleic acid sequence encoding CD8α and/or CD8β that can be operably linked to a nucleic acid encoding a tag, such as, but not limited to, wherein the nucleic acid encoding a tag is at the 5' upstream of the nucleic acid sequence encoding CD8α or CD8β such that the tag is fused to the N-terminus of CD8α or CD8β when expressed and/or wherein the tag is a CD34 enrichment tag. The isolated nucleic acid according to the current invention and the nucleic acid sequence encoding CD8α and/or CD8β can optionally be interconnected with an internal ribosome entry site or a nucleic acid sequence encoding a self-cleaving peptide, such as selected from the group consisting of P2A, E2A, F2A and T2A.

In aspects of the invention, the vector disclosed, described and/or defined herein is a viral vector and comprises one or more cell surface receptors that bind to a ligand on a target engineered cell, heterologous viral envelope glycoproteins, fusion glycoproteins, T-cell activation or co-stimulation molecules, ligands for CD19 or a functional fragment thereof, cytokines or cytokine-based transduction enhancers, and/or transmembrane proteins comprising a mitogenic domain and/or cytokine-based domain exposed on the surface and/or conjugated to the surface of the viral vector. The vector can further be pseudotyped with a Cocal or a Nipah virus envelope protein, and the Nipah envelope protein can be engineered to bind EpCAM, CD4, or CD8. In certain aspects, a vector according to the current invention comprises a lipid nanoparticle comprising ionizable cationic lipid, a sterol, a phospholipid, a non-functionalized PEG-lipid, a functionalized PED-lipid wherein the functionalized PEG-lipid has been conjugated with a binding moiety, such as wherein the vector comprises a lipid nanoparticle (LNP) comprising a PEG lipid and further comprising one or more of a phospholipid, an ionizable cationic lipid, a sterol, a co-lipid and a further PEG-lipid or combination thereof and wherein LNP comprises mRNA.

A further aspect of the invention relates to an engineered cell expressing the TCR as disclosed, described and/or defined herein and/or the isolated nucleic acid disclosed, described and/or defined herein and/or which comprises a vector disclosed, described and/or defined herein.

Optionally, the engineered cell according to the current comprises a chromosomal gene knock out of one or more TCR gene(s), one or more HLA gene(s), or both, such as wherein the engineered cell comprises a knockout of an HLA gene selected from the group consisting of an a-1-macroglobulin gene, a-2-macroglobulin gene, a-3-macroglobulin gene, b-1-microglobulin gene, b-2-microglobulin gene, and combinations thereof, or such as, wherein the engineered cell comprises a knockout of a TCR gene selected from a TCR alpha variable region gene, TCR beta variable region gene, TCR constant region gene, and combination thereof. An engineered cell according to the current invention can express CD8a and/or CD8b, optionally the CD8 alpha and/or CD8 beta is fused to a CD34 enrichment tag. The engineered cells can be enriched using the CD34 enrichment tag.

Typically, the engineered cell disclosed, described and/or defined herein, is a hematopoietic progenitor cell, peripheral blood mononuclear cell (PBMC), cord blood cell, or immune cell. In an embodiment, the immune cell is a T cell, cytotoxic lymphocyte, cytotoxic lymphocyte precursor cell, cytotoxic lymphocyte progenitor cell, cytotoxic lymphocyte stem cell, CD4+ T cell, CD8+ T cell, CD4/CD8 double negative T cell, gamma delta (gd) T cell, natural killer (NK) cell, NK-T cell, dendritic cell, or a combination thereof. In one embodiment, the cell is derived from a hematopoietic stem cell (HSC) or an induced pluripotent stem cell (iPSC). The T cell can be a naive T cell, central memory T cell, effector memory T cell, or a combination thereof, or a primary T cell or a cell of a T cell line.

In a currently preferred embodiment, the engineered cell disclosed, described and/or defined herein does not express or has a lower surface expression of an endogenous TCR and is capable of producing a cytokine or a cytotoxic molecule when contacted with a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex.

In embodiments, the engineered cell according to the current invention is capable of producing TNF-alpha, IL-2, and/or IFN-gamma, and/or a perforin and/or a granzyme, such as granzyme B. An engineered cell according to the current invention can be contacted with a target cell in vitro or ex vivo and is preferably capable of producing a higher level of cytokine or a cytotoxic molecule when contacted with a target cell with a heterozygous expression of human RAS, such as an at least 1.05-fold higher level of cytokine or a cytotoxic molecule.

In embodiments, the engineered cell according to the current invention is capable of killing a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. The killing is determined by a killing assay and the ratio of the engineered cell and the target cell in the killing assay is from 1:64 to 1:1, wherein the target cell is a target cell pulsed with a range of between 1×10³ nM and 0.1 nM of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. In particular the target cell is a cell monoallelic for HLA-A*11.

The engineered cell of the current invention does not induce T cell expansion, cytokine release, or cytotoxic killing when in contact with a target cell that presents a peptide selected from the group consisting of VVVGAGGVGK (SEQ ID NO: 3) presented in a HLA-A*11 complex, or VVGAGGVGK (SEQ ID NO: 4) presented in a HLA-A*11 complex.

In an embodiment, the engineered cell disclosed, described and/or defined herein is capable of killing a higher number of target cells when contacted with target cells with a heterozygous expression of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. Typically, the cell killing is at least 1.05-fold higher.

In aspects, the target cell is a cell line or a primary cell, such as selected from the group consisting of a cancer cell line, a primary cancer cell line, a transformed cell line, and an immortalized cell line, such as a HEK293 derived cell line.

The current invention also relates to a population of engineered cells disclosed, described and/or defined herein as well as to a pharmaceutical composition comprising any one or more of the engineered cells or vectors of the current invention, such as wherein the vectors are selected from the group consisting of viral vectors, LNPs or any other carrier comprising the nucleic acid sequence described, disclosed and/or defined herein.

### Detailed description of invention

The present invention, for the first time, discloses a T-cell receptor (TCR), which is isolated and/or expressed in an engineered cell, specifically recognizing a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) or VVGAVGVGK (SEQ ID NO:2) presented in a HLA-A*11 complex.

In one aspect, the invention relates to a method of treating a disorder characterized by G12V muted human RAS expression in a subject comprising administering to said subject a therapeutically effective amount of genetically engineered cells that express a TCR as disclosed and/or defined herein, or a pharmaceutical composition disclosed and/or defined herein, wherein said disorder is a solid or haematological malignancy expressing the peptide of VVVGAVGVGK (SEQ ID NO: 1) and/or VVGAVGVGK (SEQ ID NO: 2), wherein said engineered cell is a T-cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour or into the volume surrounding a solid tumour.

Said method is in particular intended for treating a disorder in a subject, wherein said subject carries an HLA-A*11 allele. In addition, said method can be used for treating a said subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by the TCR according to the current invention, such as wherein said subject carries a different HLA-A, HLA-B or HLA-C allele than HLA-A*11:01 , that is capable of specifically presenting the peptide of SEQ ID NO: 1 or SEQ ID NO: 2 and which is recognized by the TCR according to the current invention.

In one aspect, the invention relates to a nucleic acid encoding a TCR comprising a paired TCR alpha- and TCR beta-chain as disclosed, described and/or defined herein.

### T-cell receptor (TCR)

The current invention relates to several new T-cell receptors (TCR), which are isolated and/or expressed in an engineered cell, and which specifically recognize a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, which consists of VVVGAVGVGK (SEQ ID NO: 1) or VVGAVGVGK (SEQ ID NO:2) presented in a HLA-A*11 complex, and wherein the individual TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

The inventors of the present application have surprisingly found novel TCRs that are able to productively engage to a G12V mutated RAS 9-mer (SEQ ID NO: 2) and/or a G12V mutated RAS 10-mer (SEQ ID NO: 1) peptides in the context of HLA-A*11:01 with high specificity, wherein productive engagement of TCR with said targets triggers a signaling, cytotoxic and/or helper response in a T-cell expressing such TCR.

### RAS

It is appreciated that the G12V mutation in KRAS corresponds to similar mutations in other RAS proteins such as HRAS and NRAS. Thus, peptides comprising the G12V mutation presented in an HLA context may be derived from a mutated HRAS or mutated NRAS instead, so that the peptide in itself may not reveal whether it stems from a G12V mutated KRAS or a mutated HRAS or a mutated NRAS, regardless of whether the numbering of the substituted G->V is in the same location as long as the surrounding amino acid residues are the same. Thus, TCRs according to the present invention may equally well bind peptides derived from mutated HRAS or mutated NRAS sharing the corresponding mutation.

A TCR according to the invention does not specifically recognize a G12V mutated human RAS peptide, consisting of VVVGAVGVGK (SEQ ID NO: 1) or WGAVGVGK (SEQ ID NO: 2) when the peptide is presented in a HLA-A*11:03 complex, and does not, when expressed in an engineered cell, cause specific cytotoxic activity towards cells expressing the wild type human RAS peptide, such as VVVGAGGVGK (SEQ ID NO: 3) or VVGAGGVGK (SEQ ID NO: 4) presented in any HLA-A complex, such as, but not limited to, a HLA-A*11 complex.

Further, when expressed in an engineered cell, a TCR according to the invention does not cause specific cytotoxic activity towards cells not expressing a G12V mutated RAS peptide, such as, but not limited to, towards cells expressing the wild type human RAS peptide, such as VVVGAGGVGK (SEQ ID NO: 3) presented in a HLA-A*11 complex or VVGAGGVGK (SEQ ID NO: 4) presented in a HLA-A*11 complex.

When expressed in an engineered cell, a TCR according to the invention does not cause cytotoxic activity towards cells expressing the G12V mutated human RAS peptide, wherein the peptide consists of VVVGAVGVGK (SEQ ID NO: 1) or WGAVGVGK (SEQ ID NO: 2) which is presented in an allelic variant of the human leukocyte antigen (HLA) molecule other than an allelic variant of the human leukocyte antigen (HLA) molecule selected from the group consisting of HLA-A*11, HLA- A*68:02, HLA- B*54:01, and HLA-A*30:01 serotype. In general, when expressed in an engineered cell, a TCR according to the invention does not cause strong cytotoxic activity towards cells expressing the G12V mutated human RAS peptide, wherein the peptide consists of VWGAVGVGK (SEQ ID NO: 1) and/or WGAVGVGK (SEQ ID NO: 2) presented in a allelic variant of the human leukocyte antigen (HLA) molecule other than the HLA-A*11 serotype.

In one embodiment, as demonstrated in the experimental section, the TCR according to the current invention is a TCR, which, when expressed in an engineered cell, has at the most 4% cross-reactivity towards any somatically expressed HLA-presented peptide other than a G12V mutated human rat sarcoma (RAS) peptide, which consists of VVVGAVGVGK (SEQ ID NO: 1) and/or WGAVGVGK (SEQ ID NO: 2) and which is presented in a HLA-A*11 complex in a cross-reactivity assay.

A TCR disclosed herein typically comprises:
a. a TCR alpha chain CDR3 sequence with at least 75% identity to a TCR alpha chain CDR3 sequence selected from the group consisting of SEQ ID NO: 88, SEQ ID NO: 90, and SEQ ID NO: 98
   and
b. a TCR beta chain CDR3 sequence with at least 75% identity to a TCR beta chain CDR3 sequence selected from the group consisting of SEQ ID NO: 89, SEQ ID NO: 91, and SEQ ID NO: 99.

In embodiments, the TCR according to the current invention comprises:
c. a TCR alpha chain CDR3 sequence with a TCR alpha chain CDR3 sequence selected from the group consisting of SEQ ID NO: 88, SEQ ID NO: 90, and SEQ ID NO: 98
   and
d. a TCR beta chain CDR3 sequence with a TCR beta chain CDR3 sequence selected from the group consisting of SEQ ID NO: 89, SEQ ID NO: 91, and SEQ ID NO: 99.

In a currently preferred embodiment of the invention the TCR alpha chain variable, domain scaffold regions, and/or or CDRs comprise and/or consist of one of the sequences selected from the group consisting of SEQ ID NO: 107, SEQ ID NO: 109, and SEQ ID NO: 117, and wherein the beta chain variable, domain scaffold regions, and/or or CDRs comprise and/or consist of one of the sequences selected from the group consisting of SEQ ID NO: 108, SEQ ID NO: 110, and SEQ ID NO: 118.

In an even more preferred embodiment of the invention the TCR comprises an alpha chain paired with a beta chain, wherein such pairs are selected from the group of alpha:beta chain pairs consisting of SEQ ID NO:107 and SEQ ID NO 108; SEQ ID NO: 109 and SEQ ID NO 110; and SEQ ID NO: 117 and SEQ ID NO:118.

A TCR according to the current invention can be selected from the group comprising chimeric, humanized and/or human TCRs.

A TCR according to the invention can comprise constant regions of the alpha and beta chains which have been modified to include cysteine residues to allow covalent connection by di-sulphide bridges or comprise no covalent connections between said paired alpha and beta chains. In embodiments, the alpha and/or beta chain comprise(s) a constant region of another mammal, such as, but not limited to, a mouse chimeric constant region(s). Further, the alpha and/or beta chain can have been modified to include cysteine residues to allow di-sulphide bridges.

In aspects, the TCR comprises a TCR alpha chain a TCR beta chain which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein. In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The alpha/beta paired TCRs of the present description may have an introduced disulfide bond between their constant domains. Preferred TCRs of this type include those which have parts of a TRA constant domain sequence and a TRBI or TRB2 constant domain sequence replaced by cysteine residues, the said cysteines forming a disulfide bond between the TRA constant domain sequence and the TRBI or TRB2 constant domain sequence of the TCR. It is appreciated by those skilled in the art that there are few amino acid residues within the TRA and TRB1/TRB2 sequence chains, respectively, that may be modified to cysteine so as to form a disulfide bond.

With or without the introduced inter-chain bond mentioned above, the alpha/beta paired TCRs of the present description may have a TRA constant domain sequence and a TRB1 or TRB2 constant domain sequences, and the TRA constant domain sequence and the TRB1 or TRB2 constant domain sequences of the TCR may be linked by the native disulphide bond.

The TCR can comprise a human/mouse chimeric TCR. In this regard, the TCR can comprise a mouse constant region of an alpha chain, mouse constant region of beta chain, or both mouse constant regions. Preferably, the TCR comprises both mouse constant regions. It is appreciated by those skilled in the art that as well as replacing the constant region(s) with those from mouse, the constant domains of another mammal such as a rat, chimpanzee, gorilla, macaque or rhesus monkey may be used, which are alternative embodiments.

Alternatively or additionally, the inventive human/ mouse chimeric TCR can comprise any of the alpha chain CDR3s of the invention.

Alternatively or additionally, the inventive human/ mouse chimeric TCR can comprise any of the beta chain CDR3s of the invention.

### G12V RAS

The inventors of the present application have surprisingly found novel TCRs that are able to bind to a G12V mutated RAS 9-mer (SEQ ID NO: 2) and/or a G12V mutated RAS 10-mer (SEQ ID NO: 1) peptides in the context of HLA-A*11:01 with high affinity and specificity.

It is appreciated that the G12V mutation in KRAS corresponds to similar mutations in other RAS proteins such as HRAS and NRAS. Thus, peptides comprising the G12V mutation presented in an HLA context may be derived from a mutated HRAS or mutated NRAS instead, so that the peptide in itself may not reveal whether it stems from a G12V mutated KRAS or a mutated HRAS or a mutated NRAS, regardless of whether the numbering of the substituted G->V is in the same location as long as the surrounding amino acid residues are the same. Thus, TCRs according to the present invention may equally well bind peptides derived from mutated HRAS or mutated NRAS sharing the corresponding mutation. In such cases, the peptide consists of VVVGAVGVGK (SEQ ID NO: 1) or VVGAVGVGK (SEQ ID NO:2).

### Specifically recognizing

The current invention relates to several new T-cell receptors (TCR), which are isolated and/or expressed in an engineered cell, and which specifically recognize a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, which consists of VVVGAVGVGK (SEQ ID NO: 1) or VVGAVGVGK (SEQ ID NO:2) presented in a HLA-A*11 complex, and wherein the individual TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

A TCR according to the invention does not specifically recognize a G12V mutated human RAS peptide, consisting of VVVGAVGVGK (SEQ ID NO: 1) or WGAVGVGK (SEQ ID NO: 2) when the peptide is presented in a HLA-A*11:03 complex, and does not, when expressed in an engineered cell, cause specific cytotoxic activity towards cells expressing the wild type human RAS peptide, such as VVVGAGGVGK (SEQ ID NO: 3) or VVGAGGVGK (SEQ ID NO: 4) presented in any HLA-A complex, such as, but not limited to, a HLA-A*11 complex.

Further, when expressed in an engineered cell, a TCR according to the invention does not cause specific cytotoxic activity towards cells not expressing a G12V mutated RAS peptide, such as, but not limited to, towards cells expressing the wild type human RAS peptide, such as VVVGAGGVGK (SEQ ID NO: 3) presented in a HLA-A*11 complex or VVGAGGVGK (SEQ ID NO: 4) presented in a HLA-A*11 complex.

When expressed in an engineered cell, a TCR according to the invention does not cause cytotoxic activity towards cells expressing the G12V mutated human RAS peptide, wherein the peptide consists of VVVGAVGVGK (SEQ ID NO: 1) or WGAVGVGK (SEQ ID NO: 2) which is presented in an allelic variant of the human leukocyte antigen (HLA) molecule other than an allelic variant of the human leukocyte antigen (HLA) molecule selected from the group consisting of HLA-A*11, HLA- A*68:02, HLA- B*54:01, and HLA-A*30:01 serotype. In general, when expressed in an engineered cell, a TCR according to the invention does not cause strong cytotoxic activity towards cells expressing the G12V mutated human RAS peptide, wherein the peptide consists of VWGAVGVGK (SEQ ID NO: 1) and/or WGAVGVGK (SEQ ID NO: 2) presented in an allelic variant of the human leukocyte antigen (HLA) molecule other than the HLA-A*11 serotype.

In one embodiment, as demonstrated in the experimental section, the TCR according to the current invention is a TCR, which, when expressed in an engineered cell, has at the most 4% cross-reactivity towards any somatically expressed HLA-presented peptide other than a G12V mutated human rat sarcoma (RAS) peptide, which consists of VVVGAVGVGK (SEQ ID NO: 1) and/or WGAVGVGK (SEQ ID NO: 2) and which is presented in a HLA-A*11 complex in a cross-reactivity assay.

### Alloreactivity: "off-target" and "off-HLA" recognition by T cell.

Alloreactivity in the context of the present invention means an unintended reactivity towards a peptide-free HLA not belonging to putative HLA context for the TCR, i.e. an HLA that does not belong to HLA-A*11 supergroup and at the same time does not present a peptide. Para-reactivity in the present context means an unintended reactivity towards an HLA that does not belong to the putative HLA context but presenting the relevant peptide, i.e. in the present context an HLA not belonging to the HLA-A*11 supergroup but presenting a G12V mutated human RAS peptide, wherein the peptide consists of VVVGAVGVGK (SEQ ID NO: 1) or VVGAVGVGK (SEQ ID NO: 2).

### Strong cytotoxic activity

Strong cytotoxic activity in the current context means T cell cytotoxic activity towards a target cell population resulting in an increase in specific lysis of the target cell population that is increased more than 4.5-fold when target cells are co-cultured with T cells in a 1:1 ratio compared to a 1:64 T cell to target cell ratio.

### TCRs of the current invention

A TCR disclosed herein typically comprises:
a. a TCR alpha chain CDR3 sequence with at least 80% identity to a TCR alpha chain CDR3 sequence selected from the group consisting of SEQ ID NO: 88, SEQ ID NO: 90, and SEQ ID NO: 98,
   and
b. a TCR beta chain CDR3 sequence with at least 80% identity to a TCR beta chain CDR3 sequence selected from the group consisting of SEQ ID NO: 89, SEQ ID NO: 91, and SEQ ID NO: 99.

In embodiments, the TCR according to the current invention comprises:
c. a TCR alpha chain CDR3 sequence with a TCR alpha chain CDR3 sequence selected from the group consisting of of SEQ ID NO: 88, SEQ ID NO: 90, and SEQ ID NO: 98
   and
d. a TCR beta chain CDR3 sequence with a TCR beta chain CDR3 sequence selected from the group consisting of SEQ ID NO: 89, SEQ ID NO: 91, and SEQ ID NO: 99.

In a currently preferred embodiment of the invention the TCR alpha and beta chain variable domain scaffold regions or CDRs of the TCR comprises and/or consists of one of the sequences selected from the group consisting of SEQ ID NO: 107, SEQ ID NO: 108, SEQ ID NO: 109, SEQ ID NO: 110, SEQ ID NO: 117, and SEQ ID NO: 118.

In a preferred embodiment the TCR comprises a paired alpha and beta chain, wherein the pairs are selected from the group consisting of SEQ ID NO: 107 and SEQ ID NO: 108; SEQ ID NO: 109 and SEQ ID NO: 110; and SEQ ID NO: 117 and SEQ ID NO: 118. Such pairs may also encompass sequences having at least 80% sequence identity to said sequences; such as at least 90% sequence identity, such as at least 95% sequence identity, such as at least 98% sequence identity or more.

A TCR according to the current invention can be selected from the group comprising chimeric, humanized and/or human TCRs.

A TCR according to the invention can comprise constant regions of the alpha and beta chains which have been modified to include cysteine residues to allow covalent connection by di-sulphide bridges or comprise no covalent connections between said paired alpha and beta chains. In embodiments, the alpha and/or beta chain comprise(s) a constant region of another mammal, such as, but not limited to, a mouse chimeric constant region(s). Further, the alpha and/or beta chain can have been modified to include cysteine residues to allow di-sulphide bridges.

In aspects, the TCR comprises a TCR alpha chain a TCR beta chain which are covalently linked, such as through a linker peptide. The TCR alpha chain and/or the TCR beta chain can further be covalently linked to a moiety, and the linked moiety can comprise an affinity tag or a label. In embodiments, the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag. In embodiments, the label is a fluorochrome or a fluorophore such as a fluorescent protein. In aspects, the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.

The current invention in one aspect relates to a soluble TCR and/or a conjugated soluble TCR according to the current invention. In aspects, the TCR is a bi-specific T-cell engager (BiTE), or it is an immune mobilizing monoclonal T-cell receptor.

The TCR in one aspect TCR comprises a paired TCR alpha- and TCR beta-chain comprising a complete assembly of combinations of the following sequences:

| | SEQUENCE | SEQUENCE | SEQUENCE |
|---|---|---|---|
| SC1alpha | SEQ ID NO: 18 | SEQ ID NO: 20 | SEQ ID NO: 28 |
| CDR1alpha | SEQ ID NO: 60 | SEQ ID NO: 62 | SEQ ID NO: 70 |
| SC2alpha | SEQ ID NO: 32 | SEQ ID NO: 34 | SEQ ID NO: 42 |
| CDR2alpha | SEQ ID NO: 74 | SEQ ID NO: 76 | SEQ ID NO: 84 |
| SC3alpha | SEQ ID NO: 46 | SEQ ID NO: 48 | SEQ ID NO: 56 |
| CDR3alpha | SEQ ID NO: 88 | SEQ ID NO: 90 | SEQ ID NO: 98 |
| SC4alpha | SEQ ID NO: 7 | SEQ ID NO: 9 | SEQ ID NO: 15 |
| SC5alpha | SEQ ID NO: 104 | SEQ ID NO: 104 | SEQ ID NO: 104 |
| SC1 beta | SEQ ID NO: 19 | SEQ ID NO: 21 | SEQ ID NO: 29 |
| CDR1beta | SEQ ID NO: 61 | SEQ ID NO: 63 | SEQ ID NO: 71 |
| SC2beta | SEQ ID NO: 33 | SEQ ID NO: 35 | SEQ ID NO: 43 |
| CDR2beta | SEQ ID NO: 75 | SEQ ID NO: 77 | SEQ ID NO: 85 |
| SC3beta | SEQ ID NO: 47 | SEQ ID NO: 49 | SEQ ID NO: 57 |
| CDR3beta | SEQ ID NO: 89 | SEQ ID NO: 91 | SEQ ID NO: 99 |
| SC4beta | SEQ ID NO: 8 | SEQ ID NO: 10 | SEQ ID NO: 16 |
| SC5beta | SEQ ID NO: 105 | SEQ ID NO: 105 | SEQ ID NO: 106 |

or sequences having at least 90% such as at least 95%, at least 97% or at least 99%, or 100% identity to said respective sequences.

In one aspect the TCR comprises a paired TCR alpha- and TCR beta-chain comprising the following combination of sequences:

| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 18 | SEQ ID NO: 60 | SEQ ID NO: 32 | SEQ ID NO: 32 | SEQ ID NO: 46 | SEQ ID NO: 88 | SEQ ID NO: 7 | SEQ ID NO: 104 |
| TCR-2 alpha | SEQ ID NO: 20 | SEQ ID NO: 62 | SEQ ID NO: 34 | SEQ ID NO: 76 | SEQ ID NO: 48 | SEQ ID NO: 90 | SEQ ID NO: 9 | SEQ ID NO: 104 |
| TCR-6 alpha | SEQ ID NO: 28 | SEQ ID NO: 70 | SEQ ID NO: 42 | SEQ ID NO: 84 | SEQ ID NO: 56 | SEQ ID NO: 98 | SEQ ID NO: 15 | SEQ ID NO: 104 |
| TCR1 beta | SEQ ID NO: 19 | SEQ ID NO: 61 | SEQ ID NO: 33 | SEQ ID NO: 75 | SEQ ID NO: 47 | SEQ ID NO: 89 | SEQ ID NO: 89 | SEQ ID NO: 89 |
| TCR2 beta | SEQ ID NO: 21 | SEQ ID NO: 63 | SEQ ID NO: 35 | SEQ ID NO: 77 | SEQ ID NO: 49 | SEQ ID NO: 91 | SEQ ID NO: 10 | SEQ ID NO: 105 |
| TCR6 beta | SEQ ID NO: 29 | SEQ ID NO: 71 | SEQ ID NO: 43 | SEQ ID NO: 85 | SEQ ID NO: 57 | SEQ ID NO: 99 | SEQ ID NO: 16 | SEQ ID NO: 106 |

or sequences having at least 90% such as at least 95%, at least 97% or at least 99%, or 100% identity to said respective sequences.

In one aspect the alpha and beta chain CDRs have the following sequences:

| CDR1alpha | CDR2 alpha | CDR3 alpha | CDRIbe ta | CDR2 beta | CDR3 beta |
|---|---|---|---|---|---|
| SEQ ID NO: 60 | SEQ ID NO: 32 | SEQ ID NO: 88 | SEQ ID NO: 61 | SEQ ID NO: 75 | SEQ ID NO: 89 |
| SEQ ID NO: 62 | SEQ ID NO: 76 | SEQ ID NO: 90 | SEQ ID NO: 63 | SEQ ID NO: 77 | SEQ ID NO: 91 |
| SEQ ID NO: 70 | SEQ ID NO: 84 | SEQ ID NO: 98 | SEQ ID NO: 71 | SEQ ID NO: 85 | SEQ ID NO: 99 |

In one aspect the alpha and beta chain CDR3s have the following sequences:

| CDR3 alpha | CDR3 beta |
|---|---|
| SEQ ID NO: 88 | SEQ ID NO: 89 |
| SEQ ID NO: 90 | SEQ ID NO: 91 |
| SEQ ID NO: 98 | SEQ ID NO: 99 |

### Nucleic acid sequences

In one aspect, the invention relates to a nucleic acid encoding a TCR comprising a paired TCR alpha- and TCR beta-chain as disclosed, described and/or defined herein.

The nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be in vitro replication or in vivo replication.

A desirable method of modifying the DNA encoding the polypeptide of the description employs the polymerase chain reaction as disclosed by Saiki R K, et al. (Saiki et al., 1988). This method may be used for introducing the DNA into a suitable vector, for example by engineering in suitable restriction sites, or it may be used to modify the DNA in other useful ways as is known in the art.

### Identity vs similarity

By an amino acid sequence having an amino acid sequence at least, for example 95% identical to a reference amino acid sequence, is intended that the amino acid sequence of e.g. the TCR, or CDR3, is identical to the reference sequence, except that the amino acid sequence may include up to 5 point mutations per each 100 amino acids of the reference amino acid sequence. In other words, to obtain a TCR, or CDR3 having an amino acid sequence at least 95% identical to a reference amino acid sequence, up to 5% of the amino acids in the reference sequence may be deleted or substituted with another amino acid, or a number of amino acids up to 5% of the total amino acids in the reference sequence may be inserted into the reference sequence. These mutations of the reference sequence may occur at the amino or carboxy terminal positions of the reference amino acid sequence or anywhere between those terminal positions, interspersed either individually among amino acids in the reference sequence or in one or more contiguous groups within the reference sequence.

In the present invention, a local algorithm program is best suited to determine identity. Local algorithm programs, (such as Smith Waterman) compare a subsequence in one sequence with a subsequence in a second sequence, and find the combination of sub-sequences and the alignment of those sub-sequences, which yields the highest overall similarity score. Internal gaps, if allowed, are penalized. Local algorithms work well for comparing two multi domain proteins, which have a single domain, or just a binding site in common.

Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al, Altschul, S.F. et al (1990)). Each sequence analysis program has a default scoring matrix and default gap penalties. In general, a molecular biologist would be expected to use the default settings established by the software program used.

### Vectors

In another aspect, the invention relates to a vector comprising a nucleic acid encoding a TCR comprising a paired TCR alpha- and TCR beta-chain as disclosed, described and/or defined herein. Said vector can be selected from the group consisting of an expression vector, a coding vector, or a viral vector. In certain aspects, said vector further comprises a nucleic acid sequence encoding CD8α and/or CD8β that can be operably linked to a nucleic acid encoding a tag, such as, but not limited to, wherein the nucleic acid encoding a tag is at the 5' upstream of the nucleic acid sequence encoding CD8α or CD8β such that the tag is fused to the N-terminus of CD8α or CD8β when expressed and/or wherein the tag is a CD34 enrichment tag. The isolated nucleic acid according to the current invention and the nucleic acid sequence encoding CD8α and/or CD8β can optionally be interconnected with an internal ribosome entry site or a nucleic acid sequence encoding a self-cleaving peptide, such as selected from the group consisting of P2A, E2A, F2A and T2A.

In aspects of the invention, the vector disclosed, described and/or defined herein is a viral vector and comprises one or more cell surface receptors that bind to a ligand on a target engineered cell, heterologous viral envelope glycoproteins, fusion glycoproteins, T-cell activation or co-stimulation molecules, ligands for CD19 or a functional fragment thereof, cytokines or cytokine-based transduction enhancers, and/or transmembrane proteins comprising a mitogenic domain and/or cytokine-based domain exposed on the surface and/or conjugated to the surface of the viral vector. The vector can further be pseudotyped with a Cocal or a Nipah virus envelope protein, and the Nipah envelope protein can be engineered to bind EpCAM, CD4, or CD8. In certain aspects, a vector according to the current invention comprises a lipid nanoparticle comprising ionizable cationic lipid, a sterol, a phospholipid, a non-functionalized PEG-lipid, a functionalized PED-lipid wherein the functionalized PEG-lipid has been conjugated with a binding moiety, such as wherein the vector comprises a lipid nanoparticle (LNP) comprising a PEG lipid and further comprising one or more of a phospholipid, an ionizable cationic lipid, a sterol, a co-lipid and a further PEG-lipid or combination thereof and wherein LNP comprises mRNA.

The nucleic acids described herein can be inserted into vectors, e.g., nucleic acid expression vectors and/or targeting vectors. Accordingly, the invention provides a vector comprising any one or more of the nucleic acids of the invention.

A "vector" is any molecule or composition that has the ability to carry a nucleic acid sequence into a suitable host cell where synthesis of the encoded polypeptide can take place *in vivo.*

Typically, and preferably, a vector is a nucleic acid that has been engineered, using recombinant DNA techniques that are known in the art, to incorporate a desired nucleic acid sequence (e.g., a nucleic acid of the invention). Desirably, the vector is comprised of any type of nucleotides, including, but not limited to DNA and RNA, which can be single-stranded or double stranded, synthesized or obtained in part from natural sources, and which can contain natural, non-natural or altered nucleotides. The vectors of the invention are not naturally-occurring as a whole. However, parts of the vectors can be naturally-occurring. Preferably, the non-naturally occurring or altered nucleotides or internucleotide linkages does not hinder the transcription or replication of the vector.

The recombinant expression vector of the invention can be any suitable recombinant expression vector, and can be used to transform or transfect any suitable host. Suitable vectors include those designed for propagation and expansion or for expression or both, such as plasmids and viruses, and are well known to those skilled in the art, as are methods to construct and use them. In a preferred embodiment, the recombinant expression vectors used include a viral vector, e.g., a lentiviral vector, used to transduce a host cell during the production of a pharmaceutical preparation, as well as recombinant expression vectors designed to maintain the recombinant DNA in bacteria.

Desirably, the recombinant expression vector comprises regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. The alpha and beta paired chains of a TCR of the present invention may be encoded by nucleic acids located in separate vectors, or may be encoded by polynucleotides located in the same vector. In preferred embodiment, TCR-alpha and TCR-beta chains of the introduced TCR may be cloned into bicistronic constructs in a single vector.

Optionally, the nucleic acid sequence of the vector may be modified such that it contains codon triplets designed for optimal transcription and translation, and which are optimised for use in human cells.

The recombinant expression vector can include one or more marker genes, which act as markers of integration. Marker of integration include genes encode the likes of Green fluorescence protein (GFP), red fluorescence protein (RFP) and blue fluorescence protein (BFP).

The recombinant expression vector can include marker genes, for example the truncated CD34 (CD34t) that would mark the transduced host T cells and act as a selection marker of recovery. In a preferred embodiment, a truncated CD34t molecule is expressed alongside the TCR of interest within the human cell.

The inventive recombinant expression vectors can be designed for either transient expression, for stable expression, or for both. Also, the recombinant expression vectors can be made for constitutive expression or for inducible expression.

The recombinant expression vector can comprise a native or synthetic promoter operably linked to the nucleotide sequence encoding the TCR, polypeptide, or protein (including functional portions and functional variants thereof), or to the nucleotide sequence which is complementary to or which hybridizes to the nucleotide sequence encoding the TCR, polypeptide, or protein. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter. Preferably strong promoters are used, such as retroviral long terminal repeats (LTRs), cytomegalovirus (CMV), murine stem cell virus (MSCV) U3, phosphoglycerate kinase (PGK), P-actin, ubiquitin, elongation factor (EF)-la (Tsuji et al., 2005), and a simian virus 40 (SV40)/CD43 composite promoter (Cooper et al., 2004; Jones et al., 2009), and the spleen focus forming virus (SFFV) promoter (Joseph et al., 2008), HCMV IE1 promoter. In a preferred embodiment, the promoter is heterologous to the nucleic acid being expressed.

The present description also relates to a host cell with the integration of a functional TCR that is capable of being expressed from the introduced recombinant promoter in the host cell. In a preferred embodiment the host cell comprising a TCR of the invention is a human T-cell of the CD8+ subtype. In some embodiments, the immune cell is selected from the group consisting of a CD8+ T-cell, T-cell progenitor cell, NK cell, NK progenitor cell or differentiated stem cell. The host cells can be allogeneic, autologous or engineered. In a preferred embodiment, the host cell used for immunotherapy is an autologous human CD8+ T-cell.

The T cells of the immunotherapy can come from any source known in the art. For example, T cells can be differentiated in vitro from a hematopoietic stem cell population, or T cells can be obtained from a subject, who may or may not be the patient who it is intended to treat. T cells can be obtained from, e.g., peripheral blood mononuclear cells (PBMCs), bone marrow, lymph node tissue, cord blood, thymus tissue, tissue from a site of infection, ascites, pleural effusion, spleen tissue, and tumors. In addition, the T cells can be derived from one or more T cell lines available in the art. T cells can also be obtained from a unit of blood collected from a subject using any number of techniques known to the skilled artisan, such as {instead} density gradient centrifugation FICOLL^{™} separation and/or apheresis.

In another embodiment, the immune cells comprising a TCR of the invention are activated and/or expanded in vitro. Methods for activating and expanding T cells are known in the art. Generally, such methods include contacting PBMC or isolated T cells with a stimulatory agent and costimulatory agent, such as anti-CD3 and anti-CD28 antibodies, generally attached to a bead or other surface, in a culture medium with appropriate cytokines, such as IL-2. Anti-CD3 and anti-CD28 antibodies attached to the same bead serve as a "surrogate" antigen presenting cell (APC). One example is The Dynabeads^{®} system, a CD3/CD28 activator/stimulator system for physiological activation of human T cells.

### Engineered cells

A further aspect of the invention relates to an engineered cell expressing the TCR as disclosed, described and/or defined herein and/or the isolated nucleic acid disclosed, described and/or defined herein and/or which comprises a vector disclosed, described and/or defined herein.

Optionally, the engineered cell according to the current comprises a chromosomal gene knock out of one or more TCR gene(s), one or more HLA gene(s), or both, such as wherein the engineered cell comprises a knockout of an HLA gene selected from the group consisting of an a-1-macroglobulin gene, a-2- macroglobulin gene, a-3-macroglobulin gene, b-1-microglobulin gene, b-2-microglobulin gene, and combinations thereof, or such as, wherein the engineered cell comprises a knockout of a TCR gene selected from a TCR alpha variable region gene, TCR beta variable region gene, TCR constant region gene, and combination thereof.

An engineered cell according to the current invention can express CD8a and/or CD8b, optionally the CD8 alpha and/or CD8 beta is fused to a CD34 enrichment tag. The engineered cells can be enriched using the CD34 enrichment tag.

Typically, the engineered cell disclosed, described and/or defined herein, is a hematopoietic progenitor cell, peripheral blood mononuclear cell (PBMC), cord blood cell, or immune cell. In an embodiment, the immune cell is a T cell, cytotoxic lymphocyte, cytotoxic lymphocyte precursor cell, cytotoxic lymphocyte progenitor cell, cytotoxic lymphocyte stem cell, CD4+ T cell, CD8+ T cell, CD4/CD8 double negative T cell, gamma delta (gd) T cell, natural killer (NK) cell, NK-T cell, dendritic cell, or a combination thereof. In one embodiment, the cell is derived from a hematopoietic stem cell (HSC) or an induced pluripotent stem cell (iPSC). The T cell can be a naive T cell, central memory T cell, effector memory T cell, or a combination thereof, or a primary T cell or a cell of a T cell line.

In a currently preferred embodiment, the engineered cell disclosed, described and/or defined herein does not express or has a lower surface expression of an endogenous TCR and is capable of producing a cytokine or a cytotoxic molecule when contacted with a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex.

In embodiments, the engineered cell according to the current invention is capable of producing TNF-alpha, IL-2, and/or IFN-gamma, and/or a perforin and/or a granzyme, such as granzyme B. An engineered cell according to the current invention can be contacted with a target cell in vitro or ex vivo and is preferably capable of producing a higher level of cytokine or a cytotoxic molecule when contacted with a target cell with a heterozygous expression of human RAS, such as an at least 1.05-fold higher level of cytokine or a cytotoxic molecule.

In embodiments, the engineered cell according to the current invention is capable of killing a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. The killing is determined by a killing assay and the ratio of the engineered cell and the target cell in the killing assay is from 1:64 to 1:1, wherein the target cell is a target cell pulsed with a range of between 1×10³ nM and 0.1 nM of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. In particular the target cell is a cell monoallelic for HLA-A*11.

The engineered cell of the current invention does not induce T cell expansion, cytokine release, or cytotoxic killing when in contact with a target cell that presents a peptide selected from the group consisting of VVVGAGGVGK (SEQ ID NO: 3) presented in a HLA-A*11 complex, or VVGAGGVGK (SEQ ID NO: 4) presented in a HLA-A*11 complex.

In an embodiment, the engineered cell disclosed, described and/or defined herein is capable of killing a higher number of target cells when contacted with target cells with a heterozygous expression of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO:2) presented in a HLA-A*11 complex. Typically, the cell killing is at least 1.05-fold higher.

In aspects, the target cell is a cell line or a primary cell, such as selected from the group consisting of a cancer cell line, a primary cancer cell line, a transformed cell line, and an immortalized cell line, such as a HEK293 derived cell line.

The current invention also relates to a population of engineered cells disclosed, described and/or defined herein as well as to a pharmaceutical composition comprising any one or more of the engineered cells or vectors of the current invention, such as wherein the vectors are selected from the group consisting of viral vectors, LNPs or any other carrier comprising the nucleic acid sequence described, disclosed and/or defined herein.

The engineered cells of the invention can substantially cross-react with cells and kill cells that express a polypeptide that contains the natural amino acid sequence of the cognate peptides, such as G12V mRAS 10-mer (SEQ ID NO: 1) and 9-mer (SEQ ID NO: 2), as defined in the aspects of the description. As can be derived from the scientific literature and databases (Rammensee et al 1999), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. In an aspect, one skilled in the art would have the ability given the teachings of the description to modify the amino acid sequence of a TCR, by maintaining the known anchor residues, and would be able to determine whether such TCR variants maintain the ability to bind MHC class I or II molecules/ G12V mRAS 10-mer (SEQ ID NO: 1) and 9-mer (SEQ ID NO: 2) complexes.

In another embodiment, a soluble TCR of the invention can be produced. In this embodiment the host cell can be a cultured mammalian cell. The mammalian host cell can be an adherent cell or a suspended cell, i.e., a cell that grows in suspension. Preferred suitable host cells are from human and are known in the art, for instance HEK293 cells and ARH-77 cells. For purposes of amplifying or replicating the recombinant expression vector, the host cell is preferably a prokaryotic cell, e.g., a DH5a cell. Other eukaryotic host cells may include yeast and insect cells.

Transformation of appropriate cell hosts with a DNA construct of the present description is accomplished by well-known methods that typically depend on the type of vector used. With regard to transformation of prokaryotic host cells, see, for example, Cohen et al. (Cohen et al., 1972) and (Green and Sambrook, 2012). Electroporation is also useful for transforming and/or transfecting cells and is well known in the art for transforming yeast-cells, bacterial cells, insect cells and vertebrate cells.

Successfully transformed cells, i.e., cells that contain a DNA construct of the present description, can be identified by well-known techniques such as polymerase chain reaction (PCR). Alternatively, the presence of the protein in the supernatant can be detected using antibodies.

### Soluble TCR and/or a conjugated soluble TCR

The current invention in one aspect relates to a soluble TCR and/or a conjugated soluble TCR according to the current invention. In aspects, the TCR is a bi-specific T-cell engager (BiTE), or it is an immune mobilizing monoclonal T-cell receptor.

The invention further relates to a pharmaceutical composition comprising a soluble protein consisting of all or a functional part of a TCR as disclosed and/or defined herein and at least one pharmaceutically acceptable excipient, carrier or stabilizer.

A pharmaceutical composition of the current invention comprises a soluble TCR, a conjugated soluble TCR, a cell expressing a TCR or a composition comprising a TCR, wherein the TCR is a TCR according to the current invention. Said soluble TCR can been conjugated to a radionuclide, a chemotherapeutic agent, a toxin or another therapeutically active component, and at least one pharmaceutically acceptable excipient, carrier or stabilizer.

### Pharmaceutical compositions

A "pharmaceutical composition" is a composition suitable for administration to a human being in a medical setting. Preferably, a pharmaceutical composition is sterile and produced according to GMP guidelines.

The inventive TCRs, nucleic acids, recombinant expression vectors, and host cells (including populations thereof), can be formulated into a pharmaceutical composition. In preferred embodiment, the invention provides a pharmaceutical composition comprising any of the TCRs and functional variants thereof, nucleic acids, expression vectors, host cells (including populations thereof), and a pharmaceutically acceptable carrier. Alternatively, the pharmaceutical composition can comprise an inventive TCR material in combination with another pharmaceutically active agents or drugs, such as a chemotherapeutic agent, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

In a preferred embodiment, the pharmaceutical composition is an autologous, transduced CD8+ T-cell, infused into a patient following pre-treatment with a conditioning regime, which may include one or more of cyclophosphamide, fludarabine, decitabine or azacytidine.

The pharmaceutical composition of the present description may also include cryoprotective agents (CPAs) such as small-molecular-weight penetrating CPAs (e.g., dimethyl sulphoxide [DMSO], glycerol, ethylene glycol, and propylene glycol) and high-molecular-weight non-penetrating agents (e.g., sucrose, polyvinylpyrrolidone, and hydroxyethyl starch). In preferred embodiment the CPA is derived from DMSO.

In a preferred embodiment pharmaceutical composition is sterile and produced according to GMP guidelines.

Pharmaceutical compositions of the present description include at least one host cell expressing a TCR of the present description, in a pharmaceutically acceptable carrier.

Pharmaceutical compositions of the present description may also include pharmaceutically acceptable excipients and/ or stabilizers.

In preferred embodiment, the composition is selected for parenteral delivery or for injection/ pumping directly into a solid tumour, into a blood vessel that feeds a tumour or into the volume surrounding a solid tumour. The preparation of such pharmaceutically acceptable compositions is within the ability of one skilled in the art. In certain embodiments, buffers are used to maintain the composition at physiological pH or at a slightly lower pH, typically within a pH range of from about 5 to about 8. In certain embodiments, when parenteral administration is contemplated, the composition is in the form of a pyrogen-free, parenterally acceptable aqueous solution comprising a composition described herein, with or without additional therapeutic agents, in a pharmaceutically acceptable vehicle. In certain embodiments, the vehicle for parenteral injection is sterile distilled water in which composition described herein, with or without at least one additional therapeutic agent, is formulated as a sterile, isotonic solution, properly preserved. In certain embodiments, the preparation involves the formulation of the desired molecule with polymeric compounds (such as polylactic acid or polyglycolic acid), beads or liposomes, that provide for the controlled or sustained release of the product, which are then delivered via a depot injection. In certain embodiments, implantable drug delivery devices are used to introduce the desired molecule.

Injectable formulations are in accordance with the invention. The requirements for effective pharmaceutical carriers for injectable compositions are well-known to those of ordinary skill in the art (see, e.g., Pharmaceutics and Pharmacy Practice, J. B. Lippincott Company, Philadelphia, Pa., Banker and Chalmers, eds., pages 238-250 (1982), and ASHP Handbook on Injectable Drugs, Toissel, 4th ed., pages 622- 630 (1986)). Preferably, when administering cells, e.g., T cells, the cells are administered via injection.

Preferably, the carrier is a pharmaceutically acceptable carrier. With respect to pharmaceutical compositions, the carrier can be any of those conventionally used and is limited only by chemico-physical considerations, such as solubility and lack of reactivity with the active compound(s), and by the route of administration. The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular inventive TCR material, as well as by the particular method used to administer the inventive TCR material. Accordingly, there are a variety of suitable formulations of the pharmaceutical composition of the invention.

### Uses of the TCRs, nucleic acids and/or vectors and pharmaceutical compositions comprising the same

In one aspect, the current invention relates to a TCR disclosed, described and/or defined herein for use as a medicament. In particular, the current invention relates to a TCR disclosed, described and/or defined herein for use in the treatment of a cancer in a patient carrying HLA-A*11, comprising administering to said patient said TCR or a pharmaceutical composition comprising said TCR, wherein said cancer is a solid or haematological malignancy expressing the peptide of SEQ ID NO: 1 or SEQ ID NO: 2, and wherein said TCR or a pharmaceutical composition comprising said TCR is infused systemically into said patient or injected or pumped directly into a solid tumour, into a blood vessel that feeds a tumour or into the volume surrounding a solid tumour in said patient.

In aspects, said treatment comprises administering to said patient an engineered cell, wherein said engineered cell is a T-cell, T-cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour or into the volume surrounding a solid tumour.

Typically, the use related to herein is for treating a disorder in a subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by a TCR disclosed, described and/or defined herein, such as wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to the current invention.

In aspects, TCRs according to the present invention are used in personalised cancer immunotherapy, such as, but not limited to, being administered in combination with a vaccine. In particular the invention relates to a combination of a tumour vaccine with a TCR-T cell therapy.

### Medical Treatments

In one aspect, the invention relates to a method of treating a disorder characterized by G12V muted human RAS expression in a subject comprising administering to said subject a therapeutically effective amount of genetically engineered cells that express a TCR as disclosed and/or defined herein, or a pharmaceutical composition disclosed and/or defined herein, wherein said disorder is a solid or haematological malignancy expressing the peptide of VVVGAVGVGK (SEQ ID NO: 1) and/or VVGAVGVGK (SEQ ID NO: 2), wherein said engineered cell is a T-cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour or into the volume surrounding a solid tumour.

Said method is in particular intended for treating a disorder in a subject, wherein said subject carries an HLA-A*11 allele. In addition, said method can be used for treating a said subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by the TCR according to the current invention, such as wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to the current invention.

In another embodiment the TCRs, the nucleic acid or the expression vector of the description are used in medicine. For example, the medicament can be used in the treatment of a cancer in a patient possessing HLA-A*11:01 comprising administering to said patient said TCR or a pharmaceutical composition comprising said TCR, wherein said cancer is a solid or haematological malignancy expressing the peptide of G12V mRAS 10-mer (SEQ ID NO: 1 and 9-mer (SEQ ID NO: 2), and wherein said TCR or a pharmaceutical composition comprising said TCR is infused systemically into said patient or injected or pumped directly into a solid tumour, into a blood vessel that feeds a tumour or into the volume surrounding a solid tumour in said patient.

In aspects a pharmaceutical composition comprises a soluble protein consisting of all or a functional part of one or more TCR(s) or one or more engineered cell(s) or one or more vector(s). In aspects the invention relates to one or more compositions each comprising a soluble protein consisting of all or a functional part of one or more TCR(s) or one or more engineered cell(s) or one or more vector(s). Thus, treatment may comprise mono- or multiplexed TCR-T cell therapy. In particular the invention relates to combination therapies with 9-mer and 10-mer reactive TCRs, as well as the TCRs of the invention being included in a larger set of TCRs targeting HLA/peptides for individual patients.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of cancer in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset of the disease, or a symptom or condition thereof.

TCRs, nucleic acids and host cells of the present description, and pharmaceutical compositions thereof, may be administered to a subject in need thereof by routes known in the art, and may vary depending on the type of cancer to be treated.

TCRs of the present description may comprise a detectable label selected from the group consisting of a radionuclide, a fluorophore and biotin. TCRs of the present description may be conjugated to a therapeutically active agent, such as a radionuclide, a chemotherapeutic agent, or a toxin.

In preferred embodiments, compositions of the present description are administered to a subject using a dosing regimen of at least two administrations separated by at least 24 hours. Dosing regimens suitable for administering compositions of the present description include, for example, once a day, once every two days, and once every three days. More preferred dosing regimens include once a week, twice a week, once every other week, once a month, and twice a month. In particular embodiments, a dose escalation regimen is used, wherein a series of increasing doses is administered to a subject over a period of days, weeks or months.

Effective doses of host cells expressing TCRs of the present invention include, for example at least about 10⁴, at least about 10⁵, at least about 10⁶, at least about 10⁷, at least about 10⁸, at least about 10⁹, at least about 10¹⁰ host cells per dose. In one embodiment, host cells of the present description are administered in a dose of between about 10⁴ to about 10¹⁰ cells per dose, preferably in a dose of between about 10⁵ to about 10⁹ cells per dose. In preferred embodiments, doses are administered in a dosing regimen over the course of at least two or more dosing cycles.

### Experimental section

The present invention is further illustrated by the following non-limiting experiments.

### Figure legends

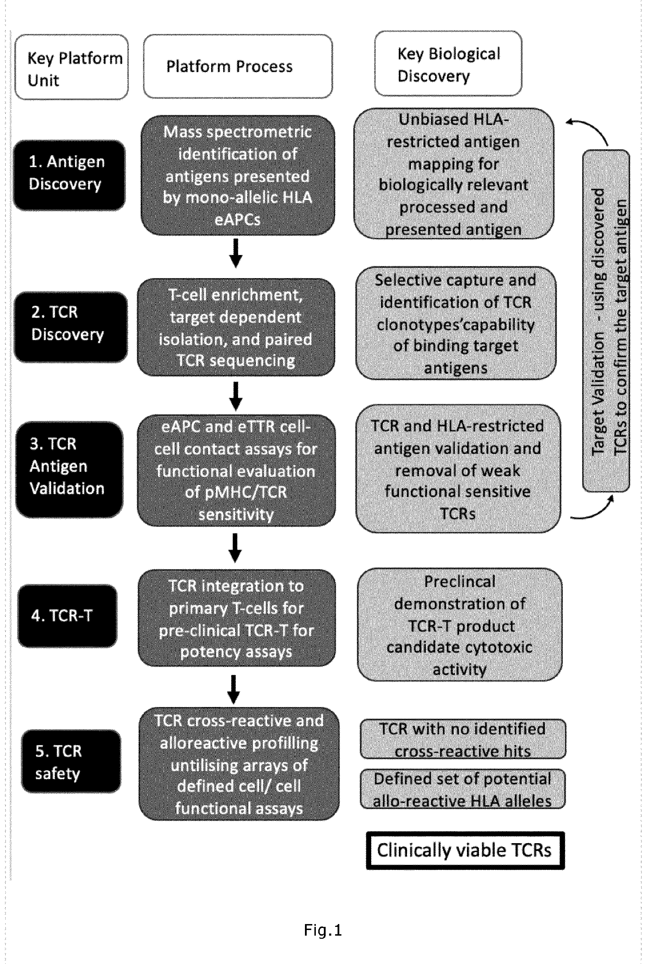

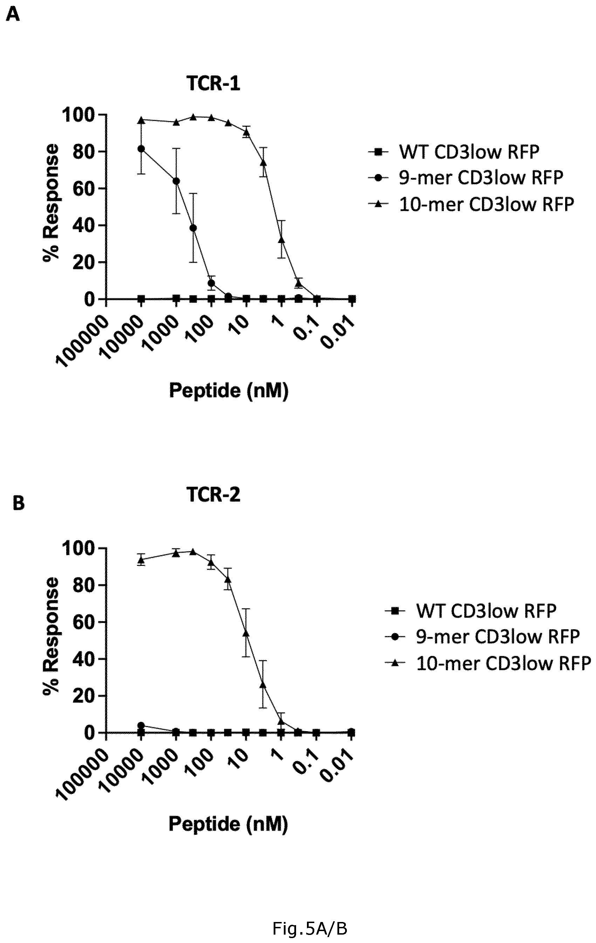

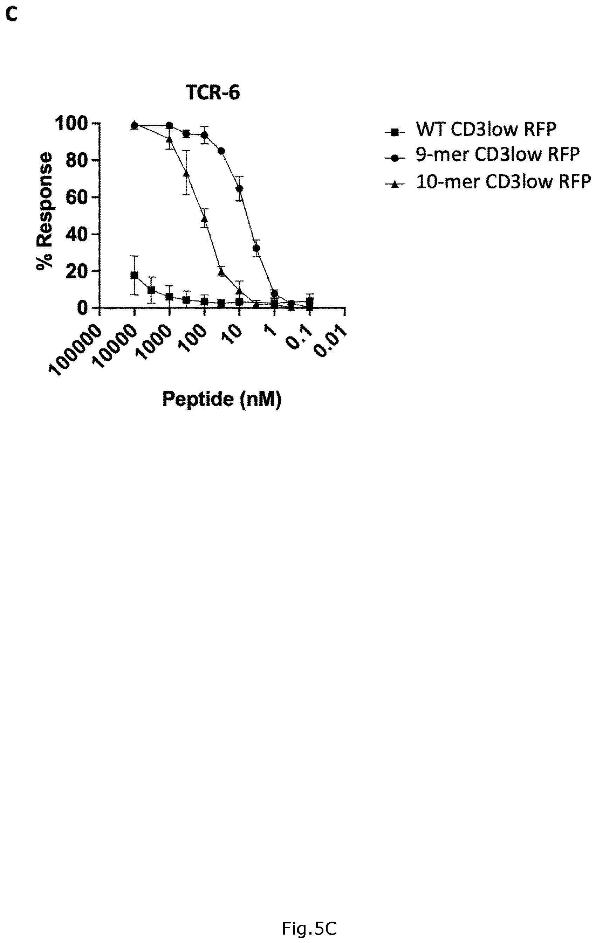

**Figure 1****: Summary of a TCR discovery platform focused on delivering clinically safe TCRs**
   The figure summarizes the Key Platform Units (left column) and the platform processes (middle column) that are used for discovery of clinically viable TCRs. A key biological discovery achieved at each step is shown (right column). Step 1. Antigen Discovery-Unbiased mass spectrometry (MS) is used for identification of peptides presented by mono-allelic HLA antigen presenting cells (eAPCs). This approach allows unbiased HLA-restricted mapping for discovery of biologically relevant, processed and presented peptides. Step 2. TCR discovery - The antigen identified in Step 1 is used for T-cells enriched, target-dependent isolation and paired TCR sequencing, allowing for selective capture and identification of TCR clonotypes' capability of binding target peptides. Step 3. TCR antigen validation - eAPC and eTPC cell contact assays are used for functional evaluation of pMHC/ TCR sensitivity. TCR and HLA-restricted peptides are validated and TCRs with low sensitivity are removed from further analysis. The discovered TCRs can in turn be used to validate the target antigen presented by the eAPC lines as well as in orthogonal cancer cell lines Step 4. TCR-T - Candidate TCRs are integrated into primary T cells and TCR-T potency assays are performed, demonstrating that the TCR-T are functional and that they possess selective cytotoxic activity. Step 5. TCR safety - the TCR discovery platform ensures that only de-risked, clinically viable TCRs progress to the clinic. Cross-reactive epitopes within the human genome are predicted from fingerprint profiles of the candidate TCRs using a computer algorithm. The identified cross-reactive hits are then presented on monoallelic eAPCs and defined cell/ cell functional contact assays are performed. TCRs with no identified cross-reactive hits are selected and a defined set of potential allo-reactive HLA alleles is determined by screening against a library of monoallalelic HLA-expressing cell lines covering 86 most common HLA haplotypes. All the above steps ensure that the end-products of the TCR discovery platform are clinically viable TCRs.

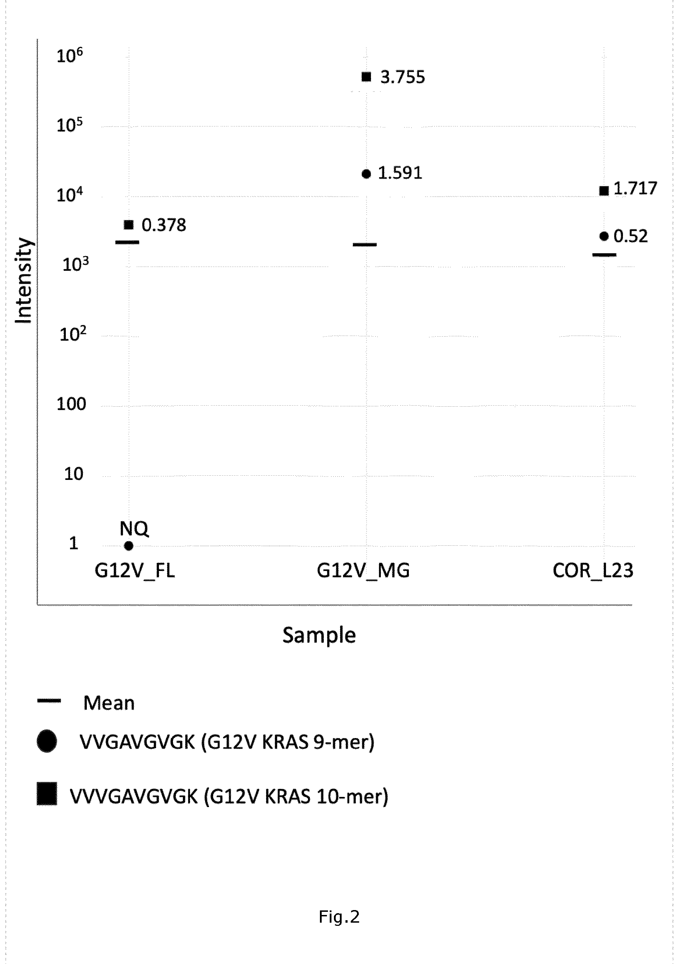

**Figure 2****: Detection of G12V mutated KRAS peptides**
   The mean intensity of peptides released from affinity purified A*11:01 pHLA complexes as measured by unbiased mass spectrometry on a Bruker TIMS-TOF2 instrument are shown, with z-scores relative to the mean (-) of specific observations of nonamer (9-mer, indicated by a full circle) and decamer (10-mer, indicated by a full square) peptides derived from G12V mutated KRAS (NQ - the particular peptides were detected but not quantified). G12V FL : A*11:01 monoallelic eAPC expressing a full length version of inactivated KRAS protein with G12V mutations (SEQ ID NO: 6); G12V MG : A*11:01 monoallelic eAPC expressing a minigene with the candidate epitope +/- up to 9 amino acids of flanking sequence (SEQ ID NO: 5). COR-L23 lung cancer cell line: cell line COR-L23 natively expressing mutated KRAS G12V and with lentivirally transduced affinity-tagged HLA-A*11:01. Intensity is plotted on Y-axis, against the sample on X-axis. The 10-mer G12V mutated KRAS peptide was observed across all three cell lines, while the 9-mer peptide was also observed in all but not quantified in the G12V_FL sample. Therefore, we have confirmed the presence of 2 G12V mutated KRAS peptides.

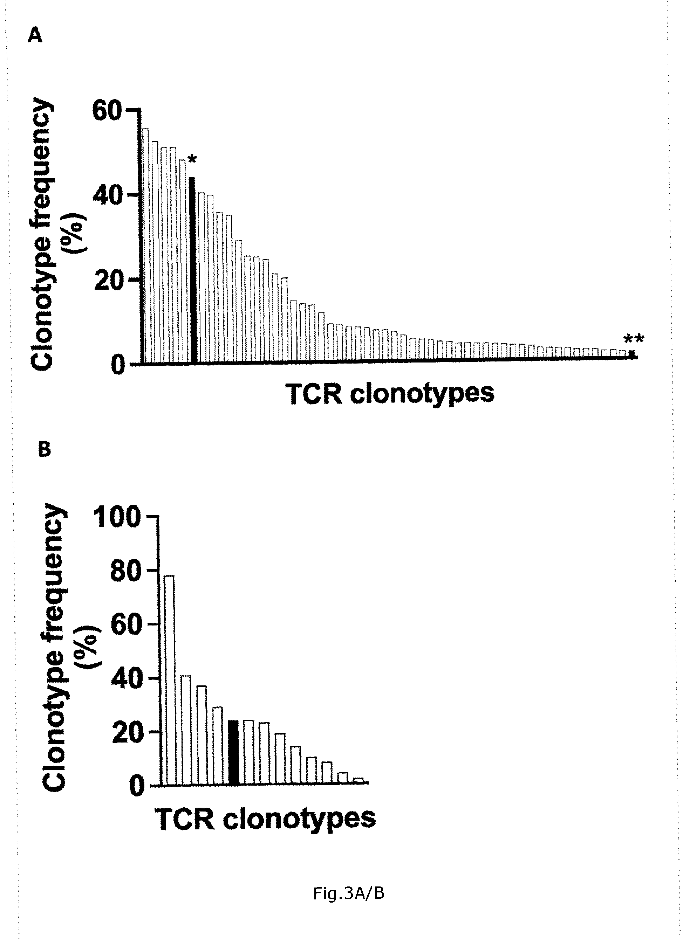

**Figure 3****: Selective expansion of TCRs specific for G12V mutated KRAS peptides.**
   Frequency distribution of TCR clonotypes enriched from G12V mutated KRAS peptide-stimulated expansions using tetramer specific for the 10-mer and 9-mer peptides. A) 10-mer specific TCR clonotypes (X axis) (n=54) are plotted against the percentage frequency each particular clonotype was observed (Y-axis). Clonotypes that were counted at least 3 times were considered enriched. Clinical candidates are shown as black bars, * TCR-1 and ** TCR-2, and have been marked retrospectively. B) A number of 9-mer specific TCR clonotypes (n=13) that were enriched at least 3 times (X axis) and are plotted against the percentage frequency that particular clonotype was observed (Y-axis). Clinical candidate, TCR-6, is shown as black bars, and has been marked retrospectively.

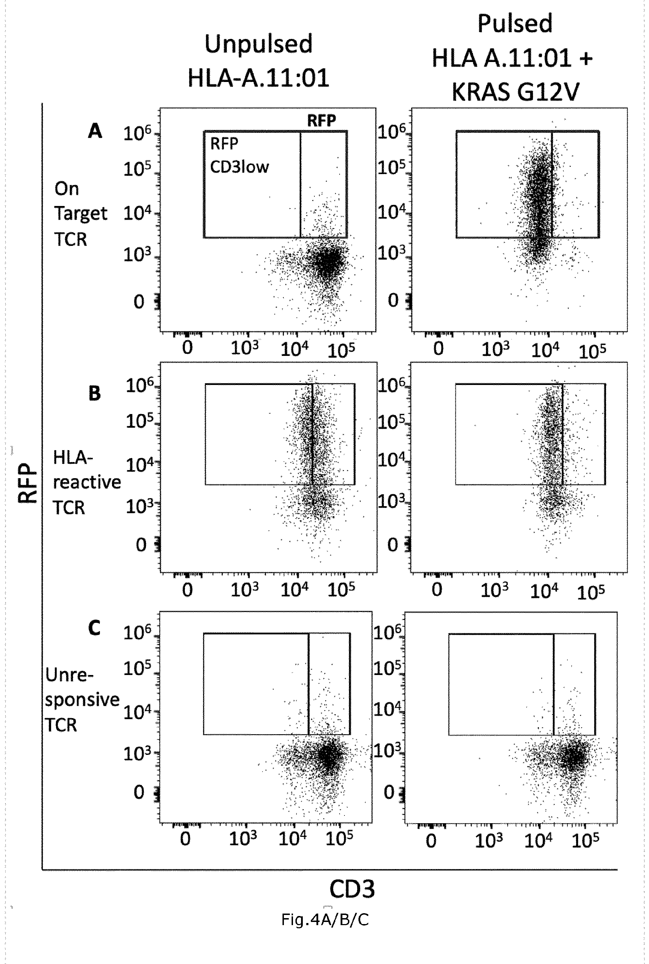

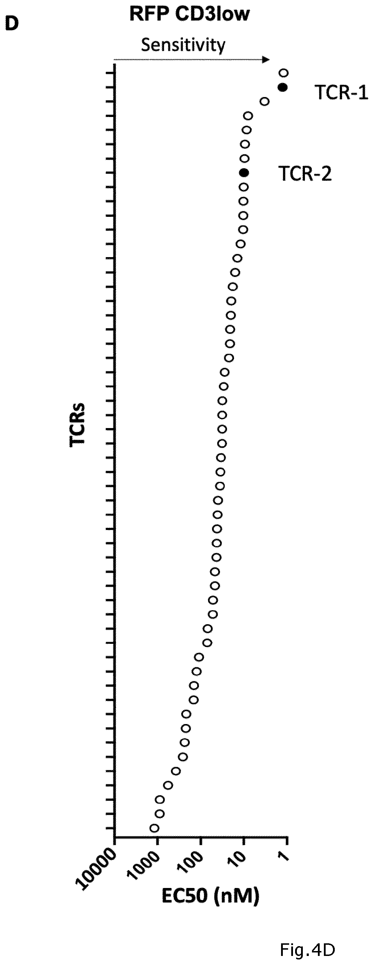

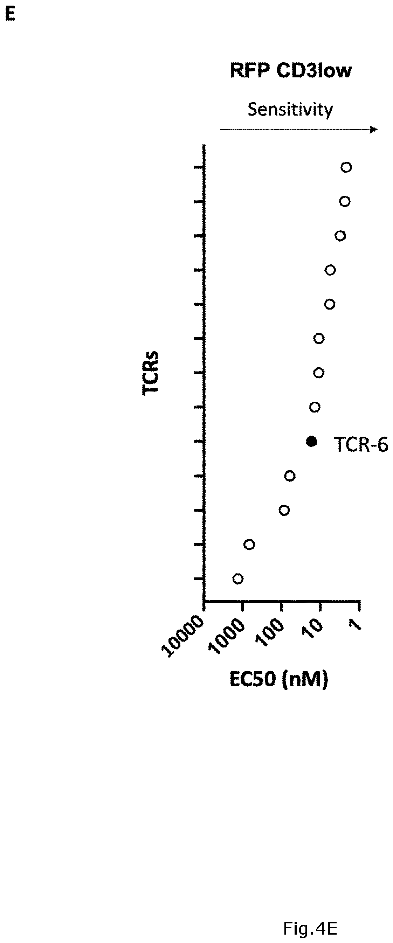

**Figure 4****: Functional evaluation of sensitivity of identified 10-mer and 9-mer G12V mutated KRAS TCRs**
   Specific activation of the engineered antigen presenting cell (eAPC) lines expressing monoallelic HLA-A*11:01 pulsed with a concentration range of the G12V mutated KRAS 10-mer or 9-mer peptides was measured by detection of the RFP signal in combination with CD3 downregulation. Figures 4 A-C show the raw data obtained during the flow cytometric analysis of RFP expression/fluorescence combined with CD3 low expression. A) is a representative of a "on target 10-mer specific TCR" unstimulated with G12V mutated KRAS 10-mer peptide (left panel) or stimulated with G12V mutated KRAS 10-mer peptide (right panel). Unstimulated eAPC HLA-A*11:01 cells stained high with the CD3 antibody and exhibited low RFP signal (Left panel). Following stimulation of the eAPC HLA-A*11:01 with the KRAS G12V 10-mer peptide, the RFP signal increased while the CD3 stain decreased (right panel). **B)** TCR that reacts with the HLA allele presented on the eAPCs, HLA-A*11 :01, without the target antigen is a representative of a "HLA-reactive TCR". The RFP/CD3 profile does not change upon stimulation with the G12V mutated KRAS 10-mer (right panel), it is always RFP positive and CD3 low. **C)** Shown here is a representative of an "unresponsive TCR" - RFP signal is low, while CD3 staining is high in both unstimulated (left panel) and stimulated cells (right panel). The flow cytometric data was used to construct sigmoidal curves from which the half maximal effective concentration (EC50) values were obtained. The sensitivity of the candidate TCRs was based on the EC50 values. **D)** EC50 (nM) distribution obtained from the RFP/CD3 low values of the G12V mutated KRAS 10-mer responsive TCRs (n=54) (X-axis) plotted against each TCR. 10-mer G12V mutated KRAS Clinical candidates TCR-1 and TCR-2 are marked by full circle, retrospectively. **E)** EC50 (nM) distribution obtained from the RFP/CD3 low values of the G12V mutated KRAS 9-mer responsive TCRs (n=13) (X-axis) against each TCR (Y-axis). 9-mer G12V mutated KRAS responsive clinical candidate TCR-6 is marked by full circle, retrospectively. During further examination of the TCR sensitivity, the ability of the clinical candidates recognizing whether the 10-mer specific TCRs can also bind the 9-mer peptides and vice versa was determined, results presented in Figure 5.
**Figure 5****: Functional evaluation of sensitivity of the clinical G12V mutated KRAS TCR candidates' pulsed with target peptides**
   Representative sigmoidal curve profiles of two 10-mer and one -9-mer specific TCR clinical candidates. The sensitivity of the 10-mer specific TCRs to the 9-mer peptide and vice versa as well as the wild type G12 KRAS peptide are compared. eTPCs expressing the TCR-1 (A) or TCR-2 (B) were co-cultured with monoallelic HLA-A*11:01-expressing cell lines pulsed with a titration range of G12V mutated KRAS 10-mer, G12V mutated KRAS 9-mer or G12 KRAS WT peptides. Percentage response (Y-Axis) of activation as measured by RFP+ CD3low is plotted against the peptide concentration in nM (X-axis). TCR-1 recognized 10-mer G12V mutated KRAS peptide (full triangle), only very weakly recognized the 9-mer G12V mutated KRAS peptide (full circle) and did not recognize the WT G12 KRAS peptide (full square). TCR-2 recognized 10-mer G12V mutated KRAS peptide (full triangle), and did not recognize the 9-mer G12V mutated KRAS peptide (full circle) or the WT G12 KRAS peptide (full square). To check the specificity of activation of the 9-mer specific TCR, eTPCs expressing the TCR-6 (C) were co-cultured with monoallelic HLA-A*11:01-expressing cell lines pulsed with a titration range of G12V 10-mer, G12V 9-mer and WT peptides. Specific activation was again measured by detection of RFP signal in combination with CD3 downregulation. TCR-6 has specificity for the 9-mer G12V mutated KRAS peptide (full circle) and to some extent the 10-mer G12V mKutated RAS peptide (full triangle) but not to the WT G12 KRAS peptide (full square). High specific sensitivity of the clinical candidates was demonstrated.

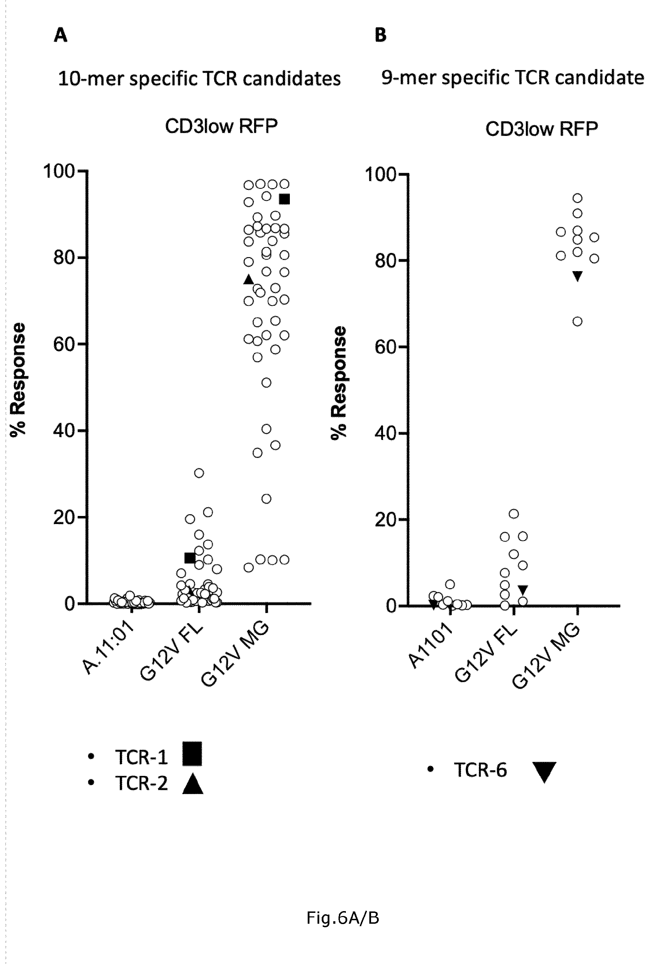

**Figure 6****: Evaluation of the clinical G12V mutated KRAS responsive 10-mer and 9-mer TCRs and constitutively expressing target peptides**
   To further validate the interaction between the 10-mer and 9-mer sensitive TCR candidates and their target peptides, we utilized the eAPCs that constitutively express the target peptides and measured the response of the eTPCs expressing the 10-mer or the 9-mer specific TCRs. **A)** Response of G12V mutated KRAS 10-mer responsive TCRs (n=54) co cultured with the monoallelic HLA-A*11:01 cell line or the monoallelic HLA-A*11:01 cell line constitutively expressing the G12V mutated KRAS epitope as a minigene (MG) (SEQ ID NO: 5) or a full length (FL) (SEQ ID NO: 6) construct. Percentage response was measured by CD3low RFP high signal. Clinical candidates are shown as black square (TCR-1) and black triangle (TCR-2). **B)** Response of G12V mutated KRAS 9-mer responsive TCRs (n=13) co cultured with the monoallelic HLA-A*11:01 cell line or the monoallelic HLA-A*11:01 cell line constitutively expressing the G12V KRAS epitope as a minigene (MG) (SEQ ID NO: 5) or a full length (FL) (SEQ ID NO: 6)construct. Percentage response was measured by CD3low staining/RFP high. Clinical candidate is shown as an upside-down triangle (TCR-6). The TCR candidates recognize and asre activated by cognate target peptide.

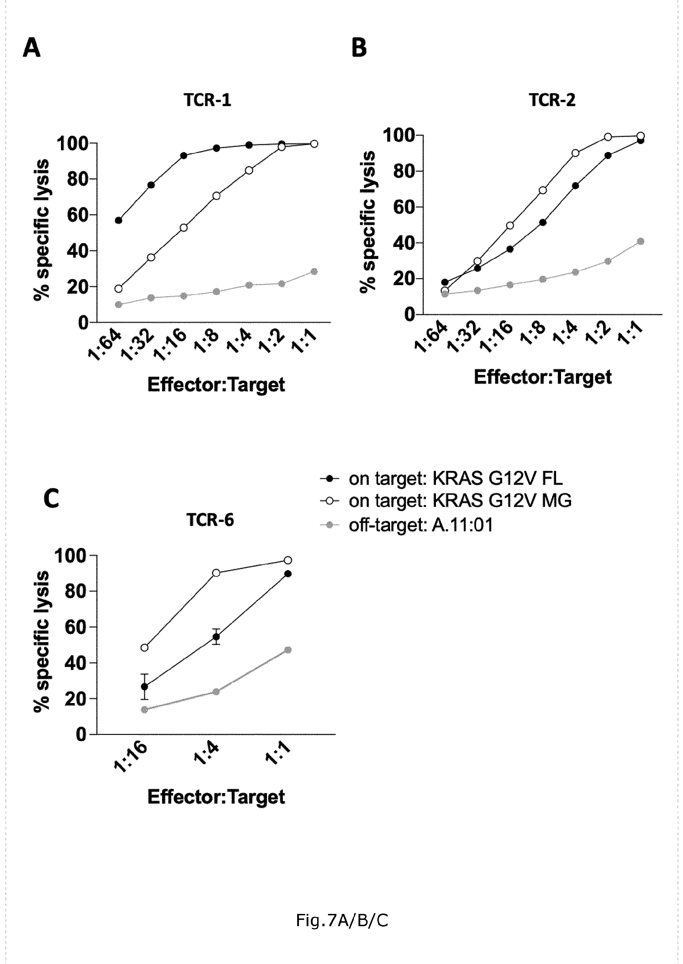

**Figure 7****: G12V mutated KRAS -specific 10-mer and 9-mer TCR clinical candidates mediate potent T cell killing of cells presenting the G12V mutated KRAS peptide in the HLA-A*11:01 context**
   Cytotoxic lysis mediated by the 10-mer sensitive TCR candidates, TCR-1 (A) and TCR-2 (B) and 9-mer sensitive TCR candidate, TCR-6 (C), as determined by Luciferase Assay. Y-axis shows % specific lysis while X-axis shows the effector (eTPC-T) to Target ratios. Target cells are monoallelic eAPCs constitutively expressing cognate HLA-A*11.01 in combination with cognate peptide G12V mutated KRAS full length (FL) (SEQ ID NO: 6) version, as well as relevant control cell lines expressing HLA-A*11:01_KRAS minigene (MG) (SEQ ID NO: 5) and HLA-A*11:01 only (used as off-target control). Specific on target lytic activity was demonstrated by TCR-1 (A), TCR-2 (B) and TCR-6 (C) with both the FL and the MG constructs. All test conditions were prepared in duplicates. Percentage killing = 100 - ((RLUtest condition)/ (RLUtarget only)*100); RLU = relative luminescence units.

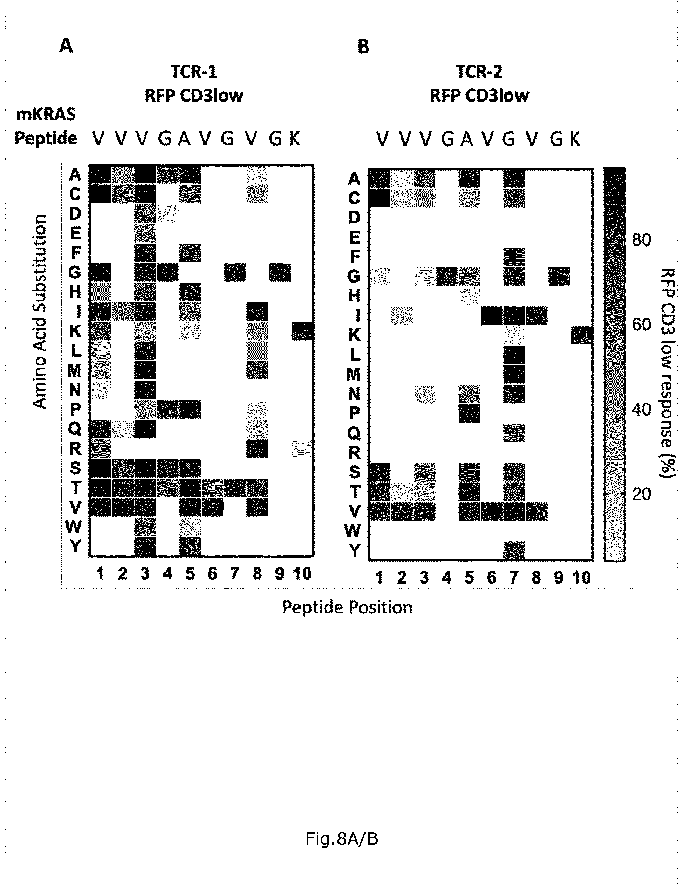

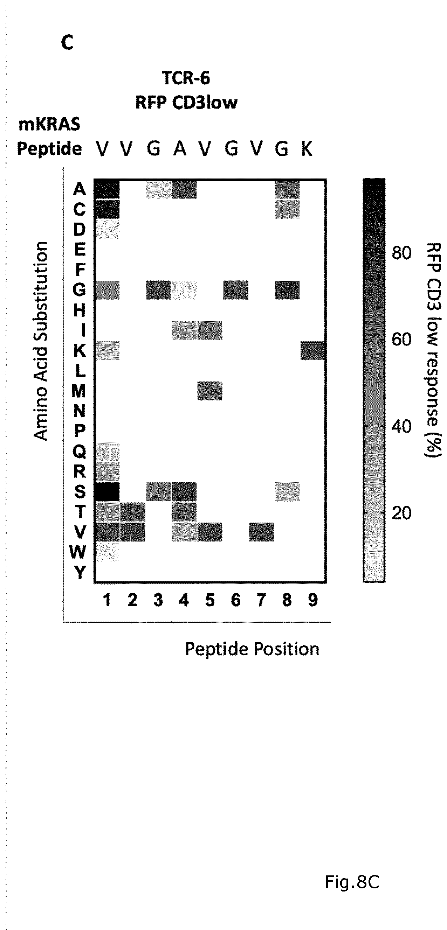

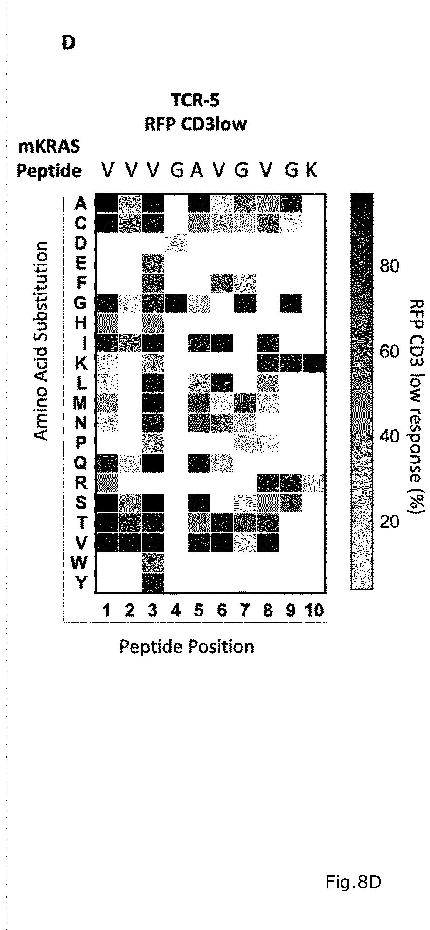

**Figure 8****: Fingerprinting profile of mutated KRAS G12V 10-mer and 9-mer sensitive TCRs**
   Fingerprinting profile of the clinical candidates. eTPC cell lines expressing the G12V mutated KRAS sensitive 10-mer or 9-mer TCRs, were stimulated with a library of monoallelic eAPCs stably expressing HLA-A*11:01 as well as the mutated version of the G12V mutated KRAS peptide. Each cell lines expressed a point mutation in a certain position. The Y-axis represents the amino acid substitution and the X-axis the peptide position where the substitution has been introduced. Activation of the eTPCs is measured by RFP/CD3low signal. **A)** Peptide scan of the KRAS G12V sensitive 10-mer TCR candidate TCR-1; **B)** Peptide scan of the KRAS G12V sensitive 10-mer TCR candidate TCR-2; **C)** Peptide scan of the KRAS G12V sensitive 9-mer TCR candidate TCR-6; **D)** Peptide scan of the KRAS G12V sensitive 10-mer TCR, TCR-5, used here as a TCR that did not pass the selection process. The darker the colour, the higher the % response due to that particular amino acid substitution in the peptide sequence. The peptide scan maps show that the TCRs are unique in their ability to recognize the target epitope and where tolerances are in that engagement.

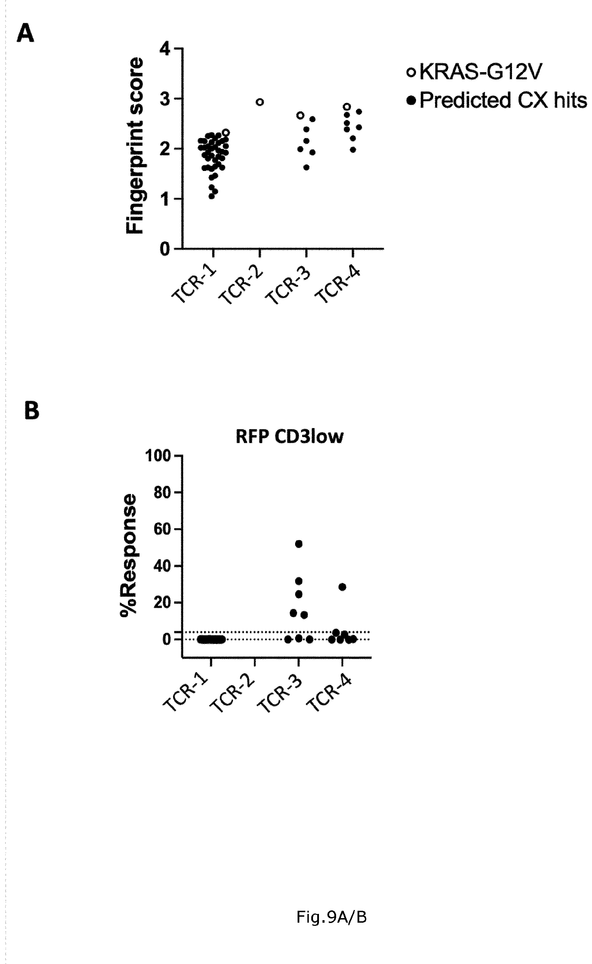

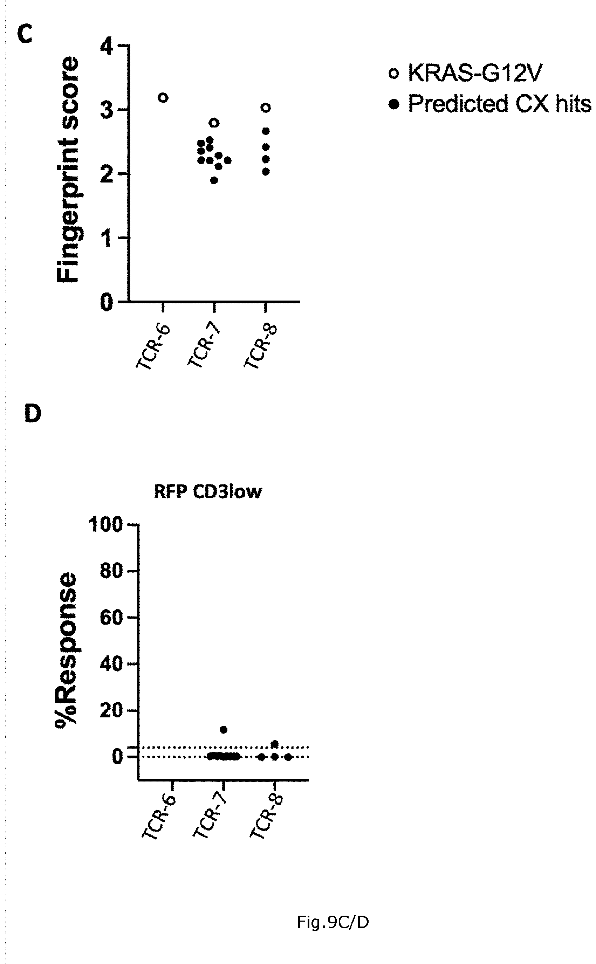

**Figure 9****: Cross-reactive profiling of the 10-mer and 9-mer sensitive G12V mutated KRAS clinical candidates**
   The fingerprint profiles from Figure 8 were analysed by a computer algorithm that generates a fingerprint score that when screened against the whole human proteome allows the prediction of cross-reactive hits. **A)** Predicted cross-reactive hits for the 10-mer sensitive TCR clinical candidates, TCR-1 and TCR-2 and two less sensitive TCRs are shown for comparison, TCR-3 and TCR-4. Based on the Fingerprint score (Y-axis), many cross-reactive hits were predicted for the clinical candidate TCR-1, while no cross-reactive hits were predicted for the TCR-2. The less sensitive TCR candidates, TCR-3 and TCR-4, showed some predicted hits based on their fingerprint score. **B)** The sequences of the predicted cross-reactive hits were subsequently used to create MG constructs that were inserted into the HLA-A*11:01 presenting cells. These cells were then contacted with the eTPC cells expressing the clinical candidate TCRs, TCR-1, TCR-2 as well as TCR-3 and TCR-4 for 18 hours. The response of the eTPCs was analysed by flow cytometry by measuring RFP CD3low signal. Although many cross-reactive hits were predicted for TCR-1, no actual cross-reactivity was detected in the cell-cell cross-reactive contact assay. TCR-2 had no predicted cross-reactive hits, therefore both clinical candidates were considered as de-risked candidates. TCR-3 and TCR-4 had predicted cross-reactive hits and few of these hits were also confirmed in the cross-reactivity cell-cell contact assay. TCRs that showed response in RFP/CD3low signal above a detectable threshold in the cell-cell contact assays were considered as cross-reactive and therefore based on these results they would be considered as unsafe for use in the clinic. Similar analysis was performed for the G12V 9-mer sensitive TCRs. **C)** Predicted cross-reactive hits for the 9-mer sensitive TCR clinical candidates, TCR-6 and two less sensitive TCRs, TCR-7 and TCR-8, are shown for comparison. Based on the Fingerprint score (Y-axis), no cross-reactive hits were predicted for the TCR-6. TCR-7 and TCR-8 showed some predicted hits based on their fingerprint score and some were also confirmed in the cell-cell cross-reactive assay **D).** TCR-3, -4, -7 and -8 have demonstrated some cross reactivity (above the 4% threshold), therefore based on these results they would be considered as unsafe for use in the clinic. TCR-1, TCR-2 and TCR-6 were selected as safe clinical candidates.

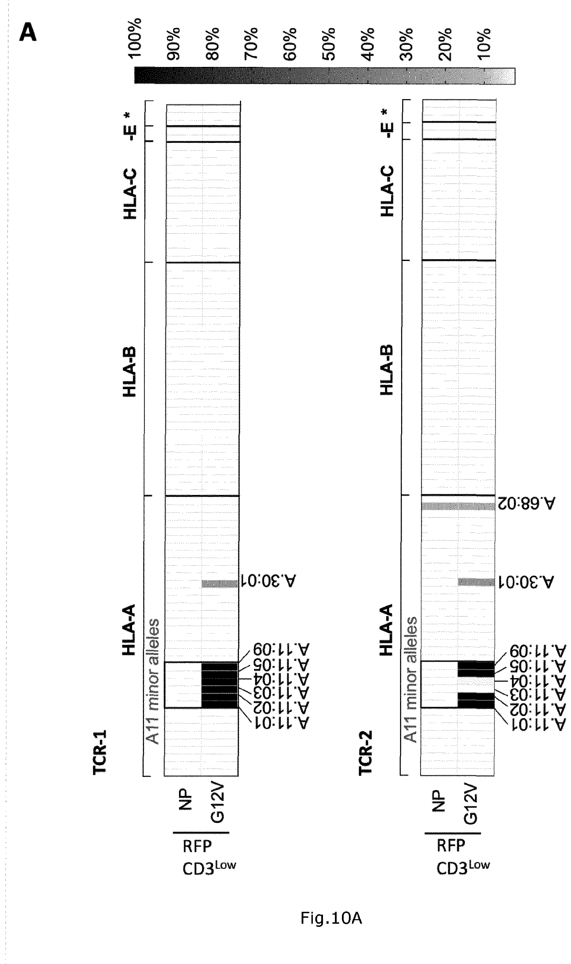

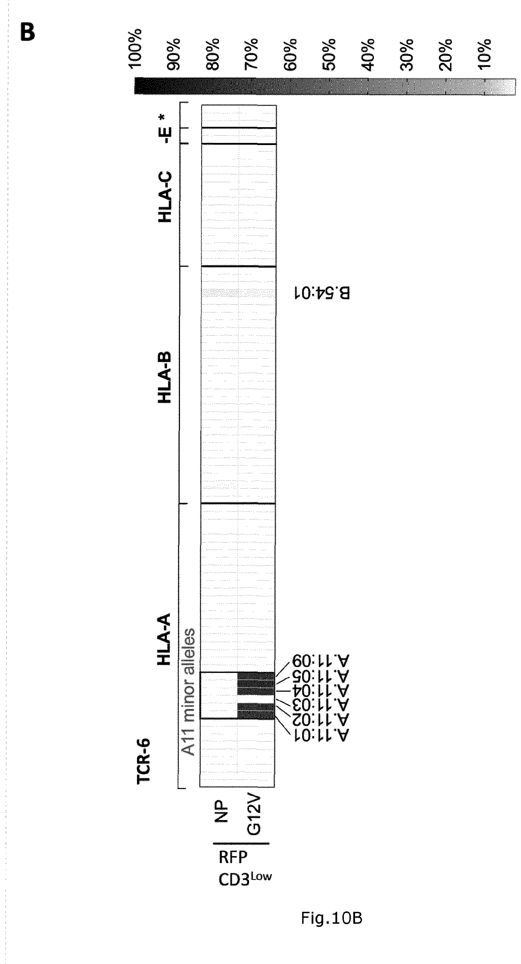

**Figure 10****: Allo and para-reactivity profile of the clinical candidates**
   To assess the alloreactivity of the clinical candidates, the responder cell lines (eTPC expressing TCR-1, TCR-2 or TCR-6 were co cultured with a library of monoallalelic HLA-expressing cell lines covering the most common haplotypes. 86 different HLA alleles were tested. The read out of the interaction was TCR activation, therefore flow cytometric analysis of RFP CD3 low signal. Para-reactivity was assessed by pulsing the library with the G12V mutated KRAS 10-mer peptide in case of TCR-1 and TCR-2, and G12V mutated KRAS 9-mer peptide in case of TCR-6. **A)** Alloreactivity and para reactivity profiles of the two G12V 10-mer clinical candidates, TCR-1 (left panel) and TCR-2 (right panel). Y-axis shows the HLA alleles tested, X-axis is the RFP/CD3low signal in the absence (NP= no peptide) or presence (G12V) of the G12V mutated KRAS peptide. TCR-1 showed no alloreactivity, and is only reactive when the peptide is added (para-reactive) to members of the HLA-A*11 group (01, 02, 03, 04, 05 and 09) and weakly para-reactive with HLA-A*30:01. TCR-2 shows alloreactivity to HLA-A*68:02 and para-reactivity to members of the HLA-A*11 group (01, 02, 05 and 09) and weakly para-reactive with HLA-A*30:01 and HLA-A*68:02. **B)** Alloreactivity and para reactivity profiles of the G12V mutated KRAS 9-mer sensitive clinical candidate, TCR-6. Y-axis shows the HLA alleles tested, X-axis is the RFPCD3low signal in the absence (NP= no peptide) or presence (G12V) of the G12V mutated KRAS 9-mer peptide. TCR-6 shows very weak alloreactivity to HLA-B*54:01 which is also detected when in the presence of the peptide. Para-reactivity to members of the HLA-A*11 group (01, 02, 04, 05 and 09) was also observed.

### Examples

The TCR discovery platform described in Figure 1, begins with the identification of HLA-restricted peptides derived from a target of interest (Step 1), which is then used in the TCR discovery (Step 2). Next, is the TCR and Antigen validation step (Step 3) where the platforms' main focus is on delivering an effective and safe clinical candidate TCR (Steps 4 and 5). The following examples describe each platform process and the key biological discovery of each step as shown in Figure 1.

### Example 1. Mass Spectrometric identification of G12V mutated KRAS peptides presented by mono-allelic HLA-A*11:01 eAPCs.

Herein is described the unbiased HLA-restricted peptide mapping for biologically relevant, processed and presented G12V mutated KRAS peptides, using mass spectrometry (MS) (Figure 2). Antigen presenting cells (eAPC) engineered to express single HLA class I allele (HLA-A*11:01) were generated as described in WO 2018/083316. The monoallelic eAPCs were transfected with either a full length (FL) version of inactivated KRAS protein with G12V mutations (G12V_FL) (SEQ ID NO: 6); or a minigene (MG) with the candidate epitope +/- up to 9 amino acids of flanking sequence (G12V_MG) (SEQ ID NO: 5),. Additionally, an orthogonal cell line (COR-L23), that natively expresses G12V mutated KRAS has been lentivirally transduced with affinity-tagged HLA-A*11:01, and was used in the assay.

Peptides released from affinity purified HLA-A*11:01 pHLA complexes were measured by unbiased mass spectrometry on a Bruker TIMS-TOF2 instrument. Figure 2 shows the z-scores relative to mean intensity of specific observations of 2 peptides derived from the G12V mutated KRAS - the nonamer (9-mer) and decamer (10-mer). Across all three cell lines, 2 epitopes of the G12V mutated KRAS were observable (these two KRAS peptide variants have been shown previously by Bear et al 2021). The z-scores in the G12V_FL sample suggest that the 10-mer G12V mutated KRAS is better processed than the 9-mer G12V mutated KRAS peptide (which was detected but not quantified). However, both peptides were detected in the G12V_MG sample and the presence of both was also confirmed in the orthogonal cell line COR-L23.

In conclusion, we confirmed 2 processed and presented G12V mutated KRAS peptides restricted to HLA-A*11:01 class I allele.

### Example 2. Selective expansion of TCR clonotypes specific for identified G12V mutated KRAS peptides.

After confirming the existence of the target peptides, the next step is to use the identified mutated KRAS G12V 10-mer and 9-mer peptides to discover TCR clonotypes that specifically bind them (Figure 1 Key platform Unit 2. TCR discovery).

To discover mutated G12V 10-mer specific TCRs, the 10-mer epitope was pulsed with monoallelic engineered antigen presenting cells (eAPCs) constitutively expressing the HLA-A*11:01. Following the peptide pulse, the eAPC cells present the 10-mer peptide on the surface in the HLA-A*11:01 context. Next, healthy, donor-derived T cells were co-cultured with the peptide pulsed monoallelic HLA-A*11:01 eAPCs. An increase in proliferation was observed upon TCR activation. To promote the growth of the activated cells, the cells were grown in the presence of interleukin -2 (IL-2). The cells were stained with an MHC multimer (a PSMA tetramer that contains the KRAS peptide), the cells were sorted by fluorescence activated cell sorting (FACS), alpha beta TCR pairs were sequenced and the frequency of appearance of each TCR clonotype was counted (Figure 3 A). If a TCR clonotype was counted at least 3 times it was considered enriched.

To discover mutated G12V 9-mer specific TCRs, the same process as described above for the 10-mer was applied. Briefly, the 9-mer peptides were pulsed with monoallelic HLA-A*11:01 eAPCs. Following the peptide pulse, the eAPC cells present the 9-mer peptide on the surface in the HLA-A*11:01 context. Next, healthy, donor-derived T cells were co-cultured with the HLA-A*11:01 monoallelic eAPCs presenting the 9-mer G12V mutated KRAS peptide. An increase in proliferation was observed upon TCR activation. To promote the growth of the activated cells, the cells were grown In the presence of interleukin -2 (IL-2). The cells were stained with an MHC multimer (PHMSA tetramer containing the KRAS peptide), the cells were FACS sorted, the TCR alpha and beta chain pairs were sequenced and the frequency of appearance of each TCR clonotype was counted (Figure 3B). If a TCR clonotype was counted at least 3 times it was considered enriched.

In conclusion, we have successfully expanded and enriched TCR clonotypes that have the capability of recognizing the 10- and 9-mer G12V mutated KRAS peptides presented by HLA-A*11:01.

### Example 3. Validation of the interaction between the identified G12V mutated KRAS-specific TCRs and HLA-A*11:01- presented G12V mutated KRAS peptides

Following the discovery of the peptides derived from the mutated KRAS and the TCRs specific for these peptides, the TCR sensitivity was determined (Figure 1, Step 3).

To measure the sensitivity of the identified 10-mer and 9-mer- specific G12V mutated KRAS TCRs, assays using cell-cell contact assays (eAPC - eTPC) with peptide-pulsed eAPCs were applied as described briefly here. Firstly, the TCRs were expressed into engineered TCR presenting cells (eTPCs) containing a synthetic reporter element that responds to TCRsp engagement (WO 2018/083317). From now on they will be referred to as responder or eTPC cells in this specification. The eTPCs have been engineered to contain a Response element that comprises of a Driver-Activator component and an Amplifier-Report component, wherein both units utilize synthetic promoters. The Driver is a synthetic promoter that is responsive to the native TCR signalling pathways, encoding three sets of tandem transcription factor binding sites for NFAT-AP1-NFkB (3xNF-AP-NB). Upon transcriptional activation, the Driver induces expression of the Activator protein, a synthetic designed transcription factor derived by fusion of the Herpes VP16 activation domain, the GAL4 DNA binding domain and two nuclear localization signals at the N- and C-terminals (NV16G4N), to which the cognate DNA recognition sequence is present 6 times in tandem in the Amplifier promoter. Both the Driver and Amplifier promoters utilize the core promoter sequence (B recognition element (BRE), TATA Box, Initiator (INR) and transcriptional start site) from HCMV IE1 promoter, immediately 3' of the respective transcription factor binding sites. The Amplifier upon transcriptional activation drives expression of the reporter, red fluorescent protein (RFP). The responder eTPCs expressing the enriched 10-mer specific G12V mutated KRAS TCRs were co-cultured with the monoallelic eAPCs presenting the HLA-A*11:01 that were either unpulsed or pulsed with a concentration gradient of either the mutated KRAS G12V 10-mer peptide, the G12V 9-mer peptide or the wild type (WT) peptide. Following the peptide pulsed cell-cell contact assays, the cells were stained CD3 antibodies and the samples were analysed for CD3 expression and RFP signal using flow cytometry . Stimulation of the TCR/CD3 complex results in RFP expression and CD3 downregulation by the eTPC, which is proportional to the strength of activation. The decrease in CD3 in combination with RFP is therefore used as a signal of TCR engagement and activation.

As an example, the flow cytometric profile of three representative 10-mer responsive TCRs is shown in Figure 4 A-C and described below. Figure 4A is a representative of a 10-mer specific TCR (on Target TCR) that is unpulsed with G12V mutated KRAS 10-mer peptide (left panel) or pulsed with G12V mutated KRAS 10-mer peptide (right panel). The unpulsed eAPC HLA-A*11:01 cells stained high with the CD3 antibody and exhibited low RFP expression. Pulsing of the eAPC HLA-A*11:01 with the G12V mutated KRAS 10-mer peptide, led to increased RFP signal and decrease in CD3 stain, thus confirming TCR activation. Figure 4B is a representative of a TCR that reacts with the HLA allele presented on the eAPCs, HLA-A*11:01, regardless of whether the target antigen is present (HLA-reactive). The RFP/CD3 profiles do not change when the cells were pulsed with the G12V mutated KRAS 10-mer, it is always RFP positive and CD3 low. Figure 4C is a representative of a TCR that is unresponsive to the mutated G12V 10-mer epitope. RFP expression is low, while CD3 staining is high in both unpulsed (left panel) and pulsed cells (right panel).

The flow cytometric data obtained from the peptide pulse assays for all the 10-mer and 9-mer responsive TCRs was used to construct sigmoidal curves from which the half maximal effective concentration (EC50) values were obtained. The data from the peptide pulse assays is represented in two ways-- Figure 4 which shows that many of the 10-mer and 9-mer responsive TCRs identified in the TCR discovery step are sensitive to the 10-mer or the 9-mer G12V mutated KRAS peptides. This is shown by plotting the TCRs against the EC50- increased sensitivity is shown with EC50 values moving towards the right. The EC50 for TCR-1 and TCR-2 were respectively, 1.26 nM (95% CI 0985 to 1.66) and 9.83 nM (95% CI 8.07 to 11.99). The EC50 of TCR-6 was 16.89 nM (95% CI 11.23 to 24.65). It was encouraging to see that many of the identified TCRs showed high sensitivity. Secondly, Figure 5 focuses on the clinical candidates and the obtained data is presented to demonstrate the sensitivity of the identified TCRs to other peptides (for example sensitivity of the 10-mer specific TCRs to the 9-mer peptide and vice versa as well as to the wild type KRAS G12 peptide (data presented Figure 5). In Figure 5 the clinical 10-mer candidate TCR, TCR-1, showed high sensitivity when incubated with eAPC cells pulsed with the G12V mutated KRAS 10-mer peptide (EC50 1.26 nM (95% CI 0985 to 1.66)), while the same TCR candidate also showed poor sensitivity when incubated with eAPC cells pulsed with the G12V mutated KRAS 9-mer peptide (EC50 421 nM (95% CI 349 to 511)). TCR-1 did not bind the WT G12 KRAS peptide (Figure 5A). The clinical 10-mer candidate TCR, TCR-2, also showed high sensitivity when incubated with eAPC cells pulsed with the G12V mutated KRAS 10-mer peptide (EC50 9.83 nM (95% CI 8.07 to 11.99)) but did not bind when incubated with eAPC cells pulsed with the G12V mutated KRAS 9-mer peptide or the WT G12 KRAS peptide (Figure 5B).

The clinical 9-mer candidate TCR, TCR-6, showed high sensitivity when incubated with eAPC cells pulsed with the G12V mutated KRAS 9-mer peptide, EC50 16.89 (95% CI 11.23 to 24.65) and detectable but lower sensitivity when incubated with eAPC cells pulsed with the G12V mutated KRAS 10-mer peptide, EC50 113nM (95% CI 99.58 to 128.5) and no interaction with the WT G12 KRAS peptide was observed (Figure 5C).

In conclusion, sensitive 10-mer and 9-mer G12V mutated KRAS responsive TCRs were identified. Furthermore, TCRs that were non-responsive upon stimulation with a mutated KRAS peptide or those TCRs that reacted to the HLA allele alone were excluded from further analysis. In the figures presented, the clinical candidates are marked retrospectively.

Next, to further to validate the interaction between the mutated KRAS G12V 10-mer and 9-mer responsive TCRs and the mutated KRAS peptides, an assay was performed to look for a response when the target peptide is endogenously expressed and presented by the antigen presenting cells, Figures 6. Therefore, monoallelic HLA-A*11:01 eAPCs or the monoallelic HLA-A*11:01 eAPC line constitutively expressing the G12V mutated KRAS peptide either as a minigene (MG)(SEQ ID NO: 5) or as a full-length epitope (FL) (SEQ ID NO: 6) were co-cultured with responder eTPCs expressing the G12V mutated KRAS 10-mer responsive TCRs (Figure 6A) or the G12V mutated KRAS 9-mer responsive TCRs (Figure 6B). The response of eTPC responder cells was measured by RFP signaling in combination with CD3 downregulation.

A partial response of the responder cells expressing the G12V mutated KRAS 10-mer responsive TCRs after incubation with the eAPC HLA-A*11:01 expressing the (SEQ ID6) was observed compared to the eAPC HLA-A*11:01 expressing the SEQ ID 5 where we see a large gradient in the response Figure 6A). This is expected as the use of the MG creates a more sensitive system because of the shorter sequence. We have also seen higher intensity detection of the peptides in the MS data (Figure 2). Only sub proportion responded to the eAPC-HLA-A*11:01 loaded with SEQ ID NO: 6 in CD3low RFP high measurements, compared to the cells loaded with the SEQ ID NO: 5. However importantly, the clinical candidates identified in the peptide pulse sensitivity assays were also detected in this experiment. Clinical candidates are marked retrospectively.

In conclusion, we show that the TCRs specific for the 10-mer and 9-mer G12V mutated KRAS TCRs recognizing HLA-A*11:01 G12V KRAS peptides, do not attack cells that express the wild type KRAS only those expressing the G12V mutation.

The overall conclusion relating to the TCR- antigen validation step within the platform is that the platform is capable of detecting sensitive TCRs, so unwanted low sensitivity TCRs can be removed, the platform is set up to differentiate between on-target TCRs, HLA-reactive TCR as well as unresponsive TCRs, and importantly the clinical candidates do not attack cells expressing the wild type KRAS peptide.

### Example 4: The G12V mutated KRAS specific TCRs mediate potent T cell killing of cells presenting the G12V mutated KRAS peptide in the HLA-A*11:01 context

As part of the clinical TCR candidate de-risking process, the cytotoxic potency of the 10-mer and 9-mer G12V mutated KRAS specific TCR candidate was analysed by using TCR-T cells co-cultured with luciferase-expressing eAPC and the ONE-Glo Luciferase Assay System (Promega) according manufacturer's instructions.

### Method

TCR-T cells (effector cells) were generated by directed endonuclease-mediated knock-in of the clinical candidate TCRs (TCR-1, TCR-2 and TCR-6) into the endogenous TRAC locus and subsequently enriched for successfully edited cells, as described in material and methods, using CD34 microbead kit (Miltenyi Biotec, according to manufacturer's instructions). Monoallelic eAPCs (Target cells, ARH-77) constitutively expressing cognate HLA-A*11.01 in combination with cognate antigen KRAS G12V FL, as well as relevant control cell lines expressing HLA-A*11:01 KRAS G12V MG and HLA-A.11:01 (off-target control, were used in cytotoxity assays with variation in effector-to-target (E:T) ratio. For variation in E:T ratio, target cells were plated at 10,000 per well in a 96-well plate and TCR-T cells were 2-fold serially diluted to obtain E:T ratios of 1:1 to 1:64. For assessing ED50 values of TCR-1, TCR-2 and TCR-6, target cells expressing HLA-A.11:01 were pulsed with varying concentrations of KRAS G12V7-16 peptide (Peptides &Elephants) in the range of 0.1pM-1µM. Upon thorough washing, peptide-pulsed target cells or no peptide control target cells were co-cultured with TCR-T cells at an E:T ratio of 1:4. All test conditions were prepared in duplicates. After 24h of co-culture, target cell killing was assessed via luciferase activity of remaining viable cells in co-cultures compared to target only control wells and calculated as: % killing = 100 - ((RLU test condition)/(RLU target only)*100); RLU = relative luminescence units.

### Results

Rapid apoptotic death of the target cells was induced by TCR-T cells expressing the TCR-1 was similar against the effector cells expressing the KRAS MG (SEQ ID NO: 5) (Figure 7A). Faster killing of target cells expressing the KRAS FL (SEQ ID NO: 6) was observed with just over 50% lysis at the highest E:T ratios (1:64). Off-target killing of HLA-A*11:01 only expressing target cells was lower, reaching below 20% at the lowest E:T ratio. TCR-2, with 50% cytolysis reached at high effector: target (1:16) ratios for the target eAPC constitutively expressing the mutated KRAS SEQ ID NO: 5 (Figure 7B). Cells constitutively expressing the KRAS (SEQ ID NO: 6) construct were killed at slightly slower kinetics, but 50% cytolysis was achieved at 1:8 effector: target ratio. At 1:1 ratio almost complete lysis of the target cells was achieved in both eAPC expressing the FL (SEQ ID NO: 6) or the MG KRAS peptide constructs. Minimal off-target lysis cell expressing only the HLA-A*11:01 was observed, less than 40% at the lowest 1:1 ratio. TCR-6 also demostrated specific on-target killing (Figure 7C).

### Conclusion

Demonstrated potent and selective toxicity of primary T cells expressing the candidate TCRs against cells presenting the G12V mutated KRAS peptide, in the HLA-A*11:01 context, with high sensitivity.

### Example 5. Cross-reactive and allo-reactive profiling of G12V mutated KRAS TCR clinical candidates.

The TCRs that passed the functional evaluation sensitivity tests are next triaged into assays that will determine their safety profile and ultimately end up with de-risked clinical candidates (Figure 1, Step 5). The first step in the safety assessment, the mutated KRAS G12V TCR 10-mer and 9-mer candidates undergo profiling for fingerprinting (Figure 8). The present example shows the fingerprinting of G12V mutated KRAS 10-mer and 9-mer TCRs using positional scanning peptide matrix. Two peptide libraries, containing all possible naturally occurring single amino acids at each position within the 9-mer (WGAVGVGK) and 10-mer (VWGAVGVGK) G12V mutated KRAS peptides, were created. Each library peptide was then cloned into antigen presenting cells to generate a library of monoallelic HLA-A*11:01 eAPCs stably expressing the mutated version of the KRAS epitope. The eAPC cells were then co-cultured for 18 hours with eTPC responder cell lines expressing the clinical candidate TCRs (TCR-1, TCR-2 and TCR-6). Activation of the responder cells was measured by RFP signal in combination with CD3 downregulation, using flow cytometry. The results are then plotted as a fingerprint profile, wherein the "y-axis" represents the amino acid introduced and the "x-axis" the position where it has been introduced. The darker the colour, the higher the response due to that particular amino acid substitution in the epitope sequence. In this example, fingerprint profiles of the 3 clinical candidates are presented (Figure 8 A-C), included is also a fingerprint profile of a failed TCR, TCR-5 (Figure 8D). The fingerprint profiles demonstrate that the TCRs are unique in their ability to recognize the target peptide and reveal where tolerances are in that TCR-target peptide engagement. Based on these tolerances, a computer algorithm was generated and used to obtain a fingerprint score. The fingerprint score is then screened against the whole human proteome to find matches to these tolerated positions and therefore predict potential cross-reactive hits (Figure 9 A and C). According to the fingerprint score, a number of cross-reactive hits were predicted for TCR-1 but no cross-reactive hits were predicted for TCR-2 (Figure 9A). Two less sensitive TCR candidates, TCR-3 and TCR-4, were included as a comparison in the prediction assay and both sho"ed p'edicted cross-reactive hits. The sequences from the predicted cross-reactive hits were then used to create minigene (MG) constructs that were introduced into the monoallelic HLA-A*11:01 eAPCs. Subsequently, the cells were co-cultured with the eTPC responder cells expressing the clinical candidates, TCR-1 and TCR-2 or the TCR-3 and TCR-4. To assess the cross-reactivity potential of the TCRs, the activation of the eTPC responder cells was measured by flow cytometry following the co-culture. A threshold of 4% was applied as a cut off for cross-reactivity and anything above this threshold was considered unsafe for clinical use. Even though TCR-1 had a number of cross-reactive hits predicted, the cross-reactivity assay has confirmed high specificity and no cross-reactive hits for either of the 10-mer clinical candidates. TCR-3 and TCR-4 have shown some cross-reactivity based on the predicted hits (Figure 9B).

Both, prediction of cross-reactive hits based on the Fingerprint score as well as the cross-reactivity assay were performed also for the 9-mer responsive TCR candidate, TCR-6 and was similarly found to be safe and non-cross-reactive. Those TCRs found to be cross-reactive were excluded from the program.

As part of the safety profile, allo-reactivity and para-reactivity were determined for the clinical candidates (Figure 10). Here, the responder eTPCs expressing either the 10-mer or the 9-mer clinical candidate TCRs were co-cultured for 18 hours with a library of monoallelic HLA-expressing eAPCs covering the most common HLA haplotypes. The response was measured by flow cytometry by detection of RFP CD3 low signal. Candidate TCR-1, showed no allo-reactivity, after pulsing the HLA eAPC library with the G12V mutated KRAS peptide, peptide-specific activation (para-reactivity) through several members of the HLA-A*11 supergroup was observed and to some extent activation through the HLA-A*30:01. Candidate TCR-2 was slightly allo-reactive with HLA-A*68:02. Para-reactivity was observed following peptide pulse assay with the G12V mutated KRAS with fewer members of the HLA-A*11 supergroup, HLA-A*30.01 and to some extent HLA-A*A68:01. Regarding the allo-reactivity of the 9-mer specific TCR candidate (Figure 10B), TCR-6 was weakly allo-reactive with HLA-B*54:01 and para-reactive with members of the HLA-A11 supergroup and to some extent with HLA-B*54:01.

In conclusion, we have identified and validated clinical candidate 10-mer and 9-mer G12V mutated KRAS specific TCRs, with no identified cross-reactive hits and a defined set of potential allo-reactive HLA alleles. With the knowledge gained from the HLA allo-reactivity assays we can proactively mitigate clinical risks and further tailor the use of the validated TCR candidates during the clinical trials depending on the HLA profile of each patient.

### List of References

Weber EW, Maus MV, Mackall CL. The Emerging Landscape of Immune Cell Therapies. Cell (2020) 181:46-62
Prior I.A., Lewis P.D., Mattos C. A comprehensive survey of ras mutations in cancer. Cancer Res. 2012;72:2457-2467
Pharmaceutics and Pharmacy Practice, J. B. Lippincott Company, Philadelphia, Pa., Banker and Chalmers, eds., pages 238-250 (1982)
ASHP Handbook on Injectable Drugs, Toissel, 4th ed., pages 622- 630 (1986)
Saiki et al., 1988
Altschul, S.F. et al (1990)
Devereux, J et al (1994)
BLAST Manual, Altschul, S.F. et al, Altschul, S.F. et al (1990)
Cooper et al., 2004;
Jones et al., 2009
Tsuji et al., 2005
Joseph et al., 2008
Cohen et al., 1972
Green and Sambrook, 2012
Rammensee et al 1999

### Items

1. A T-cell receptor (TCR), which is isolated and/or expressed in an engineered cell, specifically recognizing a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or VVGAVGVGK (SEQ ID NO:2) presented in a HLA-A*11 complex, and wherein the TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.
2. The TCR according to item 1, which does not specifically recognize a G12V mutated human RAS peptide, wherein the peptide consists of VWGAVGVGK (SEQ ID NO: 1) and/or WGAVGVGK (SEQ ID NO: 2) presented in a HLA-A*11:03 complex.
3. The TCR according to item 1 or 2, wherein the TCR, when expressed in an engineered cell, does not cause specific cytotoxic activity towards cells not expressing a G12V mutated RAS peptide.
4. The TCR according to any of items 1 to 3, wherein the TCR, when expressed in an engineered cell, does not cause specific cytotoxic activity towards cells expressing the wild type human RAS peptide, such as VVVGAGGVGK (SEQ ID NO: 3) presented in a HLA-A*11 complex and/or VVGAGGVGK (SEQ ID NO: 4) presented in a HLA-A*11 complex.
5. The TCR according to any of the preceding items, wherein the TCR, when expressed in an engineered cell, does not cause cytotoxic activity towards cells expressing the G12V mutated human RAS peptide, wherein the peptide consists of VWGAVGVGK (SEQ ID NO: 1) and/or WGAVGVGK (SEQ ID NO: 2) presented in an allelic variant of the human leukocyte antigen (HLA) molecule other than an allelic variant of the human leukocyte antigen (HLA) molecule selected from the group consisting of HLA-A*11, HLA- A*68:02, HLA- B*54:01, and HLA- A*30:01 serotype.
6. The TCR according to any of the preceding items, wherein the TCR, when expressed in an engineered cell, does not cause strong cytotoxic activity towards cells expressing the G12V mutated human RAS peptide, wherein the peptide consists of VVVGAVGVGK (SEQ ID NO: 1) and/or VVGAVGVGK (SEQ ID NO: 2) presented in an allelic variant of the human leukocyte antigen (HLA) molecule other than the HLA-A*11 serotype.
7. The TCR according to any of the preceding items, wherein the TCR, when expressed in an engineered cell, has at the most 4% cross-reactivity towards any somatically expressed HLA-presented peptide other than a G12V mutated human rat sarcoma (RAS) peptide, which consists of WVGAVGVGK (SEQ ID NO: 1) and/or VVGAVGVGK (SEQ ID NO: 2) and which is presented in a HLA-A*11 complex in a cross-reactivity assay.
8. The TCR according to any of the preceding items comprising:
a. a TCR alpha chain CDR3 sequence with at least 75% identity to a TCR alpha chain CDR3 sequence selected from the group consisting of SEQ ID NO: 88, SEQ ID NO: 90, and SEQ ID NO: 98
   and
b. a TCR beta chain CDR3 sequence with at least 80% identity to a TCR beta chain CDR3 sequence selected from the group consisting of SEQ ID NO: 89, SEQ ID NO: 91, and SEQ ID NO: 99.
9. The TCR according to any of the preceding items comprising:
a. a TCR alpha chain CDR3 sequence with a TCR alpha chain CDR3 sequence selected from the group consisting of SEQ ID NO: 89, SEQ ID NO: 91, and SEQ ID NO: 99
   and
b. a TCR beta chain CDR3 sequence with a TCR beta chain CDR3 sequence selected from the group consisting of SEQ ID SEQ ID NO: 89, SEQ ID NO: 91, and SEQ ID NO: 99.
10. The TCR according to any one of the preceding items, wherein the TCR comprises a paired TCR alpha- and TCR beta-chain comprising a complete assembly of combinations of the following sequences:

| | SEQUENCE | SEQUENCE | SEQUENCE |
|---|---|---|---|
| SC1alpha | SEQ ID NO: 18 | SEQ ID NO: 20 | SEQ ID NO: 28 |
| CDR1alpha | SEQ ID NO: 60 | SEQ ID NO: 62 | SEQ ID NO: 70 |
| SC2alpha | SEQ ID NO: 32 | SEQ ID NO: 34 | SEQ ID NO: 42 |
| CDR2alpha | SEQ ID NO: 74 | SEQ ID NO: 76 | SEQ ID NO: 84 |
| SC3alpha | SEQ ID NO: 46 | SEQ ID NO: 48 | SEQ ID NO: 56 |
| CDR3alpha | SEQ ID NO: 88 | SEQ ID NO: 90 | SEQ ID NO: 98 |
| SC4alpha | SEQ ID NO: 7 | SEQ ID NO: 9 | SEQ ID NO: 15 |
| SC5alpha | SEQ ID NO: 104 | SEQ ID NO: 104 | SEQ ID NO: 104 |
| SC1beta | SEQ ID NO: 19 | SEQ ID NO: 21 | SEQ ID NO: 29 |
| CDR1beta | SEQ ID NO: 61 | SEQ ID NO: 63 | SEQ ID NO: 71 |
| SC2beta | SEQ ID NO: 33 | SEQ ID NO: 35 | SEQ ID NO: 43 |
| CDR2beta | SEQ ID NO: 75 | SEQ ID NO: 77 | SEQ ID NO: 85 |
| SC3beta | SEQ ID NO: 47 | SEQ ID NO: 49 | SEQ ID NO: 57 |
| CDR3beta | SEQ ID NO: 89 | SEQ ID NO: 91 | SEQ ID NO: 99 |
| SC4beta | SEQ ID NO: 8 | SEQ ID NO: 10 | SEQ ID NO: 16 |
| SC5beta | SEQ ID NO: 105 | SEQ ID NO: 105 | SEQ ID NO: 106 |

or sequences having at least 90% such as at least 95%, at least 97% or at least 99%, or 100% identity to said respective sequences.
11. The TCR according to any of the preceding items, wherein the TCR comprises a paired TCR alpha- and TCR beta-chain comprising the following combination of sequences:

| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 18 | SEQ ID NO: 60 | SEQ ID NO: 32 | SEQ ID NO: 32 | SEQ ID NO: 46 | SEQ ID NO: 88 | SEQ ID NO: 7 | SEQ ID NO: 104 |
| TCR-2 alpha | SEQ ID NO: 20 | SEQ ID NO: 62 | SEQ ID NO: 34 | SEQ ID NO: 76 | SEQ ID NO: 48 | SEQ ID NO: 90 | SEQ ID NO: 9 | SEQ ID NO: 104 |
| TCR-6 alpha | SEQ ID NO: 28 | SEQ ID NO: 70 | SEQ ID NO: 42 | SEQ ID NO: 84 | SEQ ID NO: 56 | SEQ ID NO: 98 | SEQ ID NO: 15 | SEQ ID NO: 104 |
| TCR1 beta | SEQ ID NO: 19 | SEQ ID NO: 61 | SEQ ID NO: 33 | SEQ ID NO: 75 | SEQ ID NO: 47 | SEQ ID NO: 89 | SEQ ID NO: 89 | SEQ ID NO: 89 |
| TCR2 beta | SEQ ID NO: 21 | SEQ ID NO: 63 | SEQ ID NO: 35 | SEQ ID NO: 77 | SEQ ID NO: 49 | SEQ ID NO: 91 | SEQ ID NO: 10 | SEQ ID NO: 105 |
| TCR6 beta | SEQ ID NO: 29 | SEQ ID NO: 71 | SEQ ID NO: 43 | SEQ ID NO: 85 | SEQ ID NO: 57 | SEQ ID NO: 99 | SEQ ID NO: 16 | SEQ ID NO: 106 |

or sequences having at least 90% such as at least 95%, at least 97% or at least 99%, or 100% identity to said respective sequences.
12. The TCR according to any of the preceding items, wherein the alpha and beta chain CDRs have the following sequences:

| CDR1alpha | CDR2 alpha | CDR3 alpha | CDRIbe ta | CDR2 beta | CDR3 beta |
|---|---|---|---|---|---|
| SEQ ID NO: 60 | SEQ ID NO: 32 | SEQ ID NO: 88 | SEQ ID NO: 61 | SEQ ID NO: 75 | SEQ ID NO: 89 |
| SEQ ID NO: 62 | SEQ ID NO: 76 | SEQ ID NO: 90 | SEQ ID NO: 63 | SEQ ID NO: 77 | SEQ ID NO: 91 |
| SEQ ID NO: 70 | SEQ ID NO: 84 | SEQ ID NO: 98 | SEQ ID NO: 71 | SEQ ID NO: 85 | SEQ ID NO: 99 |

13. The TCR according to any of the preceding items, wherein the alpha and beta chain CDR3s have the following sequences:

| CDR3 alpha | CDR3 beta |
|---|---|
| SEQ ID NO: 88 | SEQ ID NO: 89 |
| SEQ ID NO: 90 | SEQ ID NO: 91 |
| SEQ ID NO: 98 | SEQ ID NO: 99 |

14. The TCR according to any of the preceding items, wherein the TCR alpha chain variable, domain scaffold regions, and/or or CDRs comprise and/or consist of one of the sequences selected from the group consisting of SEQ ID NO: 107, SEQ ID NO: 109, and SEQ ID NO: 117, and wherein the beta chain variable, domain scaffold regions, and/or or CDRs comprise and/or consist of one of the sequences selected from the group consisting of SEQ ID NO: 108, SEQ ID NO: 110, and SEQ ID NO: 118.
15. The TCR according to any of the preceding items, wherein the TCR is selected from the group comprising chimeric, humanized and/or human TCRs.
16. The TCR according to any of the preceding items wherein the constant regions of the alpha and beta chains have been modified to include cysteine residues to allow covalent connection by di-sulphide bridges.
17. The TCR according to any of items 1-16, wherein there are no covalent connections between said paired alpha and beta chains.
18. The TCR according to any of items 1-17, wherein the alpha and/or beta chain comprise(s) a constant region of another mammal.
19. The TCR according to item 18, wherein the alpha and/or beta chain comprise(s) a mouse chimeric constant region(s).
20. The TCR according to any of items 1- 19, wherein the alpha and/or beta chain have been modified to include cysteine residues to allow di-sulphide bridges.
21. The TCR according to any of items 1-20, wherein the TCR alpha chain and the TCR beta chain are covalently linked.
22. The TCR according to any of items 1-21, wherein the TCR alpha chain and the TCR beta chain are covalently linked through a linker peptide.
23. The TCR according to any of the preceding items wherein the TCR alpha chain and/or the TCR beta chain are covalently linked to a moiety.
24. The TCR according to item 23, wherein the covalently linked moiety comprises an affinity tag or a label,
25. The TCR according to item 24, wherein the tag is selected from the group consisting of a CD34 enrichment tag, glutathione-S-transferase (GST), calmodulin binding protein (CBP), protein C tag. Myc tag, Halo tag, HA tag, Flag tag, His tag, biotin tag, and V5 tag.
26. The TCR according to item 24, wherein the label is a fluorochrome or a fluorophore such as a fluorescent protein.
27. The TCR according to item 23, wherein the covalently linked moiety is selected from the group consisting of an inflammatory agent, cytokine, toxin, cytotoxic molecule, radioactive isotope, an enzyme that catalyses a cell wall sorting reaction, and an antibody or antigen-binding fragment thereof.
28. A soluble TCR and/or a conjugated soluble TCR, wherein the TCR is a TCR as defined in any of items 1-27.
29. The TCR according to item 28, wherein the TCR is a bi-specific T-cell engager (BiTE), or is an immune mobilizing monoclonal T-cell receptor.
30. A pharmaceutical composition comprising a soluble protein consisting of all or a functional part of a TCR as defined in any of items 1-29 and at least one pharmaceutically acceptable excipient, carrier or stabilizer.
31. A pharmaceutical composition comprising a soluble TCR, a conjugated soluble TCR, a cell expressing a TCR or a composition comprising a TCR, wherein the TCR is a TCR as defined in any of items 1-29.
32. A pharmaceutical composition comprising a soluble protein consisting of all or a functional part of a TCR as defined in any of items 1-29, wherein said soluble protein has been conjugated to a radionuclide, a chemotherapeutic agent, a toxin or another therapeutically active component, and at least one pharmaceutically acceptable excipient, carrier or stabilizer.
33. A method of treating a disorder characterized by G12V muted human RAS expression in a subject comprising administering to said subject a therapeutically effective amount of genetically engineered cells that express a TCR as defined in any of items 1-29, or a pharmaceutical composition as defined in any one of items 30-32, wherein said disorder is a solid or haematological malignancy expressing the peptide of VVVGAVGVGK (SEQ ID NO: 1) and/or VVGAVGVGK (SEQ ID NO: 2), wherein said engineered cell is a T-cell, T-cell progenitor cell, NK cell, NK progenitor cell or a differentiated stem cell and which is either autologous or allogenic, and wherein about 10⁴ to about 10¹⁰ engineered cells are infused systemically into said subject or injected or pumped directly into a solid tumour, into a blood vessel that feeds a tumour or into the volume surrounding a solid tumour.
34. The method of treating a disorder in a subject according to item 33, wherein said subject carries a HLA-A*11 allele.
35. The method of treating a disorder in a subject according to any of items 33-34, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by the TCR according to any of items 1-29.
36. The method of treating a disorder in a subject according to any of items 33-35, wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to any of items 1-29.
37. The TCR according to any of items 1-29 for use as a medicament.
38. The TCR according to any of items 1-29 for use in the treatment of a cancer in a patient carrying HLA-A*11, comprising administering to said patient said TCR or a pharmaceutical composition comprising said TCR, wherein said cancer is a solid or haematological malignancy expressing the peptide of SEQ ID NO: 1 and/or SEQ ID NO: 2, and wherein said TCR or a pharmaceutical composition comprising said TCR is infused systemically into said patient or injected or pumped directly into a solid tumour, into a blood vessel that feeds a tumour or into the volume surrounding a solid tumour in said patient.
39. The TCR for use according to item 38, for use in the treatment of a cancer in a patient carrying HLA-A*11:01, comprising administering to said patient an engineered cell, wherein said cancer is a solid or haematological malignancy expressing the peptide of SEQ ID NO: 1 or SEQ ID NO:2, wherein said engineered cell is a T-cell, T-cell progenitor cell, NK cell, NK progenitor cell or differentiated stem cell and which is either autologous or allogenic, and wherein about 10⁴ to about 10¹⁰ engineered cells are infused systemically into said patient or injected or pumped directly into a solid tumour, into a blood vessel that feeds a tumour or into the volume surrounding a solid tumour.
40. The TCR for use according to item 38 or 39, for use in treating a disorder in a subject, wherein said subject carries an alternate subform of HLA-A*11 other than HLA-A*11:03, which alternate subform of HLA-A*11 is capable of specifically presenting the peptide of SEQ ID NO: 1 or SEQ ID NO: 2 and which is recognized by the TCR according to any of items 1-29.
41. The TCR for use according to item 38 or 39, for use in treating a disorder in a subject, wherein said subject carries a different HLA-A, HLA-B or HLA-C allele than HLA-A*11:01, that is capable of specifically presenting the peptide of SEQ ID NO: 1 or SEQ ID NO: 2 and which is recognized by the TCR according to any of items 1-29.
42. A nucleic acid encoding a TCR comprising a paired TCR alpha- and TCR beta-chain as defined in any one of items 1-29.
43. A vector comprising a nucleic acid encoding a TCR comprising a paired TCR alpha- and TCR beta-chain as defined in any one of the items 1-29
44. A vector according to item 43, which is a vector selected from the group consisting of an expression vector, a coding vector, or a viral vector.
45. The vector according to item 43 or 44, wherein the vector further comprises a nucleic acid sequence encoding CD8α and/or CDBβ.
46. The vector according to item 45, wherein the nucleic acid sequence encoding CD8α or CD8β is operably linked to a nucleic acid encoding a tag.
47. The vector according to item 46, wherein the nucleic acid encoding a tag is at the 5' upstream of the nucleic acid sequence encoding CD8α or CD8β such that the tag is fused to the N-terminus of CD8α or CD8β when expressed.
48. The vector according to item 46 or 47, wherein the tag is a CD34 enrichment tag.
49. The vector according to any of items 45-48, wherein the isolated nucleic acid of item 41 and the nucleic acid sequence encoding CD8α and/or CD8β according to any of items 45-47 are interconnected with an internal ribosome entry site or a nucleic acid sequence encoding a self-cleaving peptide.
50. The vector according item 49, wherein the self-cleaving peptide is selected from the group consisting of P2A, E2A, F2A and T2A.
51. The vector according to any of items 43-50, wherein the vector is a viral vector and comprises one or more cell surface receptors that bind to a ligand on a target engineered cell, heterologous viral envelope glycoproteins, fusion glycoproteins, T-cell activation or co-stimulation molecules, ligands for CD19 or a functional fragment thereof, cytokines or cytokine-based transduction enhancers, and/or transmembrane proteins comprising a mitogenic domain and/or cytokine-based domain exposed on the surface and/or conjugated to the surface of the viral vector.
52. The vector according to any of items 43-51, wherein the vector the vector is pseudotyped with a Cocal or a Nipah virus envelope proteins.
53. The vector according item 52, wherein the Nipah envelope protein is engineered to bind EpCAM, CD4, or CD8.
54. The vector according to any of items 43-53, wherein the vector comprises a lipid nanoparticle comprising ionizable cationic lipid, a sterol, a phospholipid, a non-functionalized PEG-lipid, a functionalized PED-lipid wherein the functionalized PEG-lipid has been conjugated with a binding moiety.
55. The vector according to any of items 43-54, wherein the vector comprises a lipid nanoparticle (LNP) comprising a PEG lipid and further comprising one or more of a phospholipid, an ionizable cationic lipid, a sterol, a co-lipid and a further PEG-lipid or combination thereof and wherein LNP comprises mRNA.
56. An engineered cell expressing the TCR as defined in any of items 1-29.
57. An engineered cell which comprises and/or expresses the isolated nucleic acid of item 42, or a vector according to any of items 43-50.
58. The engineered cell according to item 56 or 57, wherein the engineered cell comprises a chromosomal gene knock out of one or more TCR gene(s), one or more HLA gene(s), or both.
59. The engineered cell according to any of items 56-58, wherein the engineered cell comprises a knockout of an HLA gene selected from the group consisting of an alpha-1-macroglobulin gene, alpha-2-macroglobulin gene, alpha-3-macroglobulin gene, beta-1-microglobulin gene, beta-2-microglobulin gene, and combinations thereof.
60. The engineered cell according to any of items 56-59, wherein the engineered cell comprises a knockout of a TCR gene selected from a TCR alpha variable region gene, TCR beta variable region gene, TCR constant region gene, and combination thereof.
61. The engineered cell according to any of items 56-60, wherein the engineered cell expresses CD8a and/or CD8b, optionally the CD8 alpha and/or CD8 beta is fused to a CD34 enrichment tag.
62. The engineered cell according to any of items 56-61, wherein the engineered cells are enriched using the CD34 enrichment tag.
63. The engineered cell according to any of items 56-62, wherein the engineered cell is a hematopoietic progenitor cell, peripheral blood mononuclear cell (PBMC), cord blood cell, or immune cell.
64. The engineered cell according to item 64, wherein the immune cell is a T cell, cytotoxic lymphocyte, cytotoxic lymphocyte precursor cell, cytotoxic lymphocyte progenitor cell, cytotoxic lymphocyte stem cell, CD4+ T cell, CD8+ T cell, CD4/CD8 double negative T cell, gamma delta (gd) T cell, natural killer (NK) cell, NK-T cell, dendritic cell, or a combination thereof.
65. The engineered cell according to item 63 or 64 wherein the cell is derived from a hematopoietic stem cell (HSC) or an induced pluripotent stem cell (iPSC).
66. The engineered cell according to item 64 or 65, wherein the T cell is ale T cell, central memory T cell, effector memory T cell, or a combination thereof.
67. The engineered cell according to any of items 64 to 66, wherein the T cell is a primary T cell or a cell of a T cell line.
68. The engineered cell according to any of items 64-66, wherein the T cell does not express or has a lower surface expression of an endogenous TCR.
69. The engineered cell according to any of items 56-68, wherein the engineered cell is capable of producing a cytokine or a cytotoxic molecule when contacted with a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or VVGAVGVGK (SEQ ID NO: 2) presented in a HLA-A*11 complex.
70. The engineered cell according to item 69, wherein, wherein the engineered cell is contacted with the target cell in vitro or ex vivo.
71. The engineered cell according to item 69 or 70, wherein the cytokine is TNF-alpha, IL-2, and/or IFN-gamma.
72. The engineered cell according to any of items 69-71, wherein the cytotoxic molecule is a perforin and/or a granzyme, such as granzyme B.
73. The engineered cell according to any of items 69-71, wherein the engineered cell is capable of producing a higher level of cytokine or a cytotoxic molecule when contacted with a target cell with a heterozygous expression of human RAS.
74. The engineered cell according to item 73, wherein the engineered cell is capable of producing an at least 1.05-fold higher level of cytokine or a cytotoxic molecule.
75. The engineered cell according to any of items 56-74, wherein the engineered cell is capable of killing a target cell that presents a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or VVGAVGVGK (SEQ ID NO: 2) presented in a HLA-A*11 complex.
76. The engineered cell according to item 75, wherein the killing is determined by a killing assay.
77. The engineered cell according to item 75 or 76, wherein the ratio of the engineered cell and the target cell in the killing assay is from 1:64 to 1:1.
78. The engineered cell according to any of items 75-77, wherein the target cell is a target cell pulsed with a range of between 1×10³ nM and 0.1 nM of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or WGAVGVGK (SEQ ID NO: 2) presented in a HLA-A*11 complex.
79. The engineered cell according to item 78, wherein the target cell is a cell monoallelic for HLA-A*11.
80. The engineered cell according to any of items 75-79, wherein the engineered cell is capable of killing a higher number of target cells when contacted with target cells with a heterozygous expression of a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/or VVGAVGVGK (SEQ ID NO: 2) presented in a HLA-A*11 complex.
81. The engineered cell according to item 80, wherein the cell killing is at least 1.05-fold higher.
82. The engineered cell according to any of items 56-81, wherein the target cell is a cell line or a primary cell.
83. The engineered cell according to item 82, wherein the target cell is selected from the group consisting of a cancer cell line, a primary cancer cell line, a transformed cell line, and an immortalized cell line.
84. The engineered cell according to item 83, wherein the target cell is a HEK293 derived cell line.
85. The engineered cell according to any of items 56-84, wherein the engineered cell does not induce T cell expansion, cytokine release, or cytotoxic killing when in contact with a target cell that presents a peptide selected from the group consisting of WVGAGGVGK (SEQ ID NO: 3) presented in a HLA-A*11 complex, or VVGAGGVGK (SEQ ID NO: 4) presented in a HLA-A*11 complex.
86. A population of engineered cells according to any one of items 57-85.
87. A pharmaceutical composition comprising any one or more of the engineered cells or vectors according to any of items 43 to 86.
88. The pharmaceutical composition according to item 87, wherein the vectors are selected from the group consisting of viral vectors, LNPs or any other carrier comprising the nucleic acid sequence according to item 42.
89**. A pharmaceutical composition comprising a soluble protein consisting of all or a functional part of one or more TCR(s) as defined in any of claims 1-29, or** one or more vector(s) according to any one of claims 42 to 55, or one or more engineered cell(s) according to any one of claims 56 to 85.

## Claims

1. A T-cell receptor (TCR), which is isolated and/or expressed in an engineered cell, specifically recognizing a G12V mutated human rat sarcoma (RAS) peptide, wherein the peptide is a mutated human Kirsten RAS (KRAS) peptide, a mutated human Harvey RAS (HRAS) peptide, or a human neuroblastoma RAS (NRAS) peptide, wherein the peptide consists of VVVGAVGVGK (SEQ ID NO: 1) and/ or VVGAVGVGK (SEQ ID NO:2) presented in a HLA-A*11 complex, and wherein the TCR comprises a paired TCR alpha- and TCR beta-chain, wherein the TCR alpha chain variable domain comprises SC1alpha-CDR1alpha-SC2alpha-CDR2alpha-SC3alpha-CDR3alpha-SC4alpha-SC5alpha, and the TCR beta chain variable domain comprises SC1beta-CDR1beta-SC2 beta-CDR2 beta-SC3beta-CDR3beta-SC4beta-SC5beta, wherein SC is a scaffold region and CDR is a complementarity determining region.

2. The TCR according to claim 1, which does not specifically recognize a G12V mutated human RAS peptide, wherein the peptide consists of VWGAVGVGK (SEQ ID NO: 1) and/ or VVGAVGVGK (SEQ ID NO: 2) presented in a HLA-A*11:03 complex.

3. The TCR according to claim 1 or 2, wherein the TCR, when expressed in an engineered cell, does not cause specific cytotoxic activity towards cells not expressing a G12V mutated RAS peptide.

4. The TCR according to any of claims 1 to 3, wherein the TCR, when expressed in an engineered cell, does not cause specific cytotoxic activity towards cells expressing the wild type human RAS peptide, such as VVVGAGGVGK (SEQ ID NO: 3) presented in a HLA-A*11 complex and/or VVGAGGVGK (SEQ ID NO: 4) presented in a HLA-A*11 complex.

5. The TCR according to any of the preceding claims, wherein the TCR, when expressed in an engineered cell, does not cause cytotoxic activity towards cells expressing the G12V mutated human RAS peptide, wherein the peptide consists of VWGAVGVGK (SEQ ID NO: 1) and/ or VVGAVGVGK (SEQ ID NO: 2) presented in an allelic variant of the human leukocyte antigen (HLA) molecule other than an allelic variant of the human leukocyte antigen (HLA) molecule selected from the group consisting of HLA-A*11, HLA- A*68:02, HLA- B*54:01, and HLA- A*30:01 serotype.

6. The TCR according to any of the preceding claims, wherein the TCR, when expressed in an engineered cell, does not cause strong cytotoxic activity towards cells expressing the G12V mutated human RAS peptide, wherein the peptide consists of VVVGAVGVGK (SEQ ID NO: 1) and/or VVGAVGVGK (SEQ ID NO: 2) presented in an allelic variant of the human leukocyte antigen (HLA) molecule other than the HLA-A*11 serotype.

7. The TCR according to any of the preceding claims, wherein the TCR, when expressed in an engineered cell, has at the most 4% cross-reactivity towards any somatically expressed HLA-presented peptide other than a G12V mutated human rat sarcoma (RAS) peptide, which consists of VVVGAVGVGK (SEQ ID NO: 1) and/or VVGAVGVGK (SEQ ID NO: 2) and which is presented in a HLA-A*11 complex in a cross-reactivity assay.

8. The TCR according to any of the preceding claims comprising:
a. a TCR alpha chain CDR3 sequence with at least 75% identity to a TCR alpha chain CDR3 sequence selected from the group consisting of SEQ ID NO: 88, SEQ ID NO: 90, and SEQ ID NO: 98
and
b. a TCR beta chain CDR3 sequence with at least 75% identity to a TCR beta chain CDR3 sequence selected from the group consisting of SEQ ID NO: 89, SEQ ID NO: 91, and SEQ ID NO: 99.

9. The TCR according to any of the preceding claims comprising:
a. a TCR alpha chain CDR3 sequence with a TCR alpha chain CDR3 sequence selected from the group consisting of SEQ ID NO: 89, SEQ ID NO: 91, and SEQ ID NO: 99
and
b. a TCR beta chain CDR3 sequence with a TCR beta chain CDR3 sequence selected from the group consisting of SEQ ID SEQ ID NO: 89, SEQ ID NO: 91, and SEQ ID NO: 99.

10. The TCR according to any one of the preceding claims, wherein the TCR comprises a paired TCR alpha- and TCR beta-chain comprising a complete assembly of combinations of the following sequences:
| | SEQUENCE | SEQUENCE | SEQUENCE |
|---|---|---|---|
| SC1alpha | SEQ ID NO: 18 | SEQ ID NO: 20 | SEQ ID NO: 28 |
| CDR1alpha | SEQ ID NO: 60 | SEQ ID NO: 62 | SEQ ID NO: 70 |
| SC2alpha | SEQ ID NO: 32 | SEQ ID NO: 34 | SEQ ID NO: 42 |
| CDR2alpha | SEQ ID NO: 74 | SEQ ID NO: 76 | SEQ ID NO: 84 |
| SC3alpha | SEQ ID NO: 46 | SEQ ID NO: 48 | SEQ ID NO: 56 |
| CDR3alpha | SEQ ID NO: 88 | SEQ ID NO: 90 | SEQ ID NO: 98 |
| SC4alpha | SEQ ID NO: 7 | SEQ ID NO: 9 | SEQ ID NO: 15 |
| SC5alpha | SEQ ID NO: 104 | SEQ ID NO: 104 | SEQ ID NO: 104 |
| SC1beta | SEQ ID NO: 19 | SEQ ID NO: 21 | SEQ ID NO: 29 |
| CDR1beta | SEQ ID NO: 61 | SEQ ID NO: 63 | SEQ ID NO: 71 |
| SC2beta | SEQ ID NO: 33 | SEQ ID NO: 35 | SEQ ID NO: 43 |
| CDR2beta | SEQ ID NO: 75 | SEQ ID NO: 77 | SEQ ID NO: 85 |
| SC3beta | SEQ ID NO: 47 | SEQ ID NO: 49 | SEQ ID NO: 57 |
| CDR3beta | SEQ ID NO: 89 | SEQ ID NO: 91 | SEQ ID NO: 99 |
| SC4beta | SEQ ID NO: 8 | SEQ ID NO: 10 | SEQ ID NO: 16 |
| SC5beta | SEQ ID NO: 105 | SEQ ID NO: 105 | SEQ ID NO: 106 |
or sequences having at least 90% such as at least 95%, at least 97% or at least 99%, or 100% identity to said respective sequences.

11. The TCR according to any of the preceding claims, wherein the TCR comprises a paired TCR alpha- and TCR beta-chain comprising the following combination of sequences:
| TCR# | SC1 | CDR1 | SC2 | CDR2 | SC3 | CDR3 | SC4 | SC5 |
|---|---|---|---|---|---|---|---|---|
| TCR-1 alpha | SEQ ID NO: 18 | SEQ ID NO: 60 | SEQ ID NO: 32 | SEQ ID NO: 32 | SEQ ID NO: 46 | SEQ ID NO: 88 | SEQ ID NO: 7 | SEQ ID NO: 104 |
| TCR-2 alpha | SEQ ID NO: 20 | SEQ ID NO: 62 | SEQ ID NO: 34 | SEQ ID NO: 76 | SEQ ID NO: 48 | SEQ ID NO: 90 | SEQ ID NO: 9 | SEQ ID NO: 104 |
| | | | | | | | | |
| TCR-6 alpha | SEQ ID NO: 28 | SEQ ID NO: 70 | SEQ ID NO: 42 | SEQ ID NO: 84 | SEQ ID NO: 56 | SEQ ID NO: 98 | SEQ ID NO: 15 | SEQ ID NO: 104 |
| TCR1 beta | SEQ ID NO: 19 | SEQ ID NO: 61 | SEQ ID NO: 33 | SEQ ID NO: 75 | SEQ ID NO: 47 | SEQ ID NO: 89 | SEQ ID NO: 89 | SEQ ID NO: 89 |
| TCR2 beta | SEQ ID NO: 21 | SEQ ID NO: 63 | SEQ ID NO: 35 | SEQ ID NO: 77 | SEQ ID NO: 49 | SEQ ID NO: 91 | SEQ ID NO: 10 | SEQ ID NO: 105 |
| TCR6 beta | SEQ ID NO: 29 | SEQ ID NO: 71 | SEQ ID NO: 43 | SEQ ID NO: 85 | SEQ ID NO: 57 | SEQ ID NO: 99 | SEQ ID NO: 16 | SEQ ID NO: 106 |
or sequences having at least 90% such as at least 95%, at least 97% or at least 99%, or 100% identity to said respective sequences.

12. The TCR according to any of the preceding claims, wherein the alpha and beta chain CDRs have the following sequences:
| CDR1alpha | CDR2 alpha | CDR3 alpha | CDRIbe ta | CDR2 beta | CDR3 beta |
|---|---|---|---|---|---|
| SEQ ID NO: 60 | SEQ ID NO: 32 | SEQ ID NO: 88 | SEQ ID NO: 61 | SEQ ID NO: 75 | SEQ ID NO: 89 |
| SEQ ID NO: 62 | SEQ ID NO: 76 | SEQ ID NO: 90 | SEQ ID NO: 63 | SEQ ID NO: 77 | SEQ ID NO: 91 |
| SEQ ID NO: 70 | SEQ ID NO: 84 | SEQ ID NO: 98 | SEQ ID NO: 71 | SEQ ID NO: 85 | SEQ ID NO: 99 |

13. The TCR according to any of the preceding claims, wherein the alpha and beta chain CDR3s have the following sequences:
| CDR3 alpha | CDR3 beta |
|---|---|
| SEQ ID NO: 88 | SEQ ID NO: 89 |
| SEQ ID NO: 90 | SEQ ID NO: 91 |
| SEQ ID NO: 98 | SEQ ID NO: 99 |

14. An engineered cell expressing a TCR as defined in any one of claims 1-13, wherein the engineered cell is selected from the group consisting of an immune cell derived from, a peripheral blood mononuclear cell (PBMC), a cord blood cell, a hematopoietic progenitor cell, an induced pluripotent stem cell, and a primary immune cell collected from any bodily tissue.

15. A nucleic acid encoding a TCR comprising a paired TCR alpha- and TCR beta-chain as defined in any one of claims 1-13.

16. A vector comprising a nucleic acid encoding a TCR comprising a paired TCR alpha- and TCR beta-chain as defined in any one of the claims 1-13.

17. A vector according to claim 16, which is a vector selected from the group consisting of an expression vector, a coding vector, or a viral vector, wherein said vector the ability to carry said nucleic acid sequence into a suitable host cell where synthesis of the encoded polypeptide takes place in vivo.

18. A pharmaceutical composition comprising a soluble protein consisting of all or a functional part of one or more TCR(s) as defined in any of claims 1-13, or one or more engineered cell(s) according to claim 14 or one or more vector(s) according to claims 16 or 17.

19. The TCR according to any of claims 1-13, an engineered cell according to claim 14, a vector according to claims 16 or 17 or a pharmaceutical composition according to claim 18 for use in the treatment of a cancer in a patient carrying HLA-A*11, comprising administering to said patient said TCR, engineered cell, vector and/or a pharmaceutical composition comprising said TCR, wherein said cancer is a solid or haematological malignancy expressing the peptide of SEQ ID NO: 1 and/ or SEQ ID NO: 2, and wherein said TCR, engineered cell, vector and/or a pharmaceutical composition comprising said TCR is infused systemically into said patient or injected or pumped directly into a solid tumour, into a blood vessel that feeds a tumour or into the volume surrounding a solid tumour in said patient.
